# FASCICULE DE BREVET EUROPEEN

(11) **EP 3 515 206 B1**
(45) Date de publication et mention de la délivrance du brevet: **05.08.2020**
(21) Numéro de dépôt: 17776972.6
(22) Date de dépôt: 19.09.2017
(51) Int. Cl.: A23L 2/02, A23L 2/78

(54) **JUS DE CANNEBERGE DÉSACIDIFIÉ ET PROCÉDÉ DE PRÉPARATION DE CE DERNIER**
ENTSÄUERTER CRANBERRY-SAFT UND VERFAHREN ZUR HERSTELLUNG DAVON
DEACIDIFIED CRANBERRY JUICE AND METHOD FOR THE PRODUCTION THEREOF

(30) Priorité: 20.09.2016 FR 1658802
(43) Date de publication de la demande: 31.07.2019
(73) Titulaire: West Invest S.A., 1941 Luxembourg (LU)
(72) Inventeur: REYNAUD, Eric, 1208 LUXEMBOURG (LU); DUVAL, Charles, 13430 EYGUIERES (FR); BAUDOUIN, Stanislas, 17180 PERIGNY (FR); MEURISSE, Jacques, 38240 MEYLAN (FR)
(74) Mandataire: Agasse, Stéphane
(86) Numéro de dépôt international: PCT/EP2017/073641
(87) Numéro de publication internationale: WO 2018/054904

(56) Documents cités:
- WO-A1-2010/121203
- US-A1- 2010 009 049
- BAZINET LAURENT ET AL: "Evolution of cranberry juice physico-chemical parameters during phenolic antioxidant enrichment by electrodialysis with filtration membrane", SEPARATION AND PURIFICATION TECHNOLOGY, vol. 87, 23 novembre 2011 (2011-11-23), pages 31-39, XP028890689, ISSN: 1383-5866, DOI: 10.1016/J.SEPPUR.2011.11.017
- SERRE ELODIE ET AL: "Deacidification of cranberry juice by electrodialysis: Impact of membrane types and configurations on acid migration and juice physicochemical characteristics", SEPARATION AND PURIFICATION TECHNOLOGY, ELSEVIER SCIENCE, AMSTERDAM, NL, vol. 163, 26 février 2016 (2016-02-26), pages 228-237, XP029466444, ISSN: 1383-5866, DOI: 10.1016/J.SEPPUR.2016.02.044
- VERA E ET AL: "Comparison of different methods for deacidification of clarified passion fruit juice", JOURNAL OF FOOD ENGINEERING, BARKING, ESSEX, GB, vol. 59, 2003, pages 361-367, XP002990783, ISSN: 0260-8774, DOI: 10.1016/S0260-8774(02)00495-8

## Description

### DOMAINE TECHNIQUE DE L'INVENTION

L'invention concerne un jus de canneberge désacidifié, un procédé de désacidification du jus, ainsi que diverses utilisations du jus désacidifié obtenu par le procédé.

La canneberge est une proche parente du bleuet nord-américain, de la myrtille européenne et de diverses autres baies du genre *Vaccinium.* Toutes ces plantes ont en commun d'être naines et rampantes, de pousser dans des sols acides et de donner des baies qui sont particulièrement riches en antioxydants.

La baie de canneberge est un fruit de 10 à 20 mm de diamètre, d'une couleur rouge vif à maturité. Sa saveur est très caractéristique car elle présente une forte acidité et astringence.

La canneberge est réputée pour ses bienfaits pour la santé et la prévention contre certaines maladies.

Les bienfaits pour la santé de la canneberge s'expliquent notamment par le fait que cette plante renferme différents types de flavonoïdes, qui participent à la prévention de l'apparition de maladies telles que les maladies cardiovasculaires, certains cancers et diverses maladies liées au vieillissement.

Les principaux flavonoïdes de la canneberge sont :
- les anthocyanes (également appelés « anthocyanosides » ou « anthocyanines ») qui confèrent la coloration rouge caractéristique du fruit ;
- les anthocyanidols (également appelés « anthocyanidines ») ;
- les flavanols, et notamment :
   les flavan-3-ol monomères (par exemple la catéchine, l'épicatéchine, la gallocatéchine, et l'épigallocatéchine) ;
   les flavan-3-ols polymères (par exemple les proanthocyanidols (également appelés « tanins condensés » ou « proanthocyanidines ») ;
- les gallotanins et ellagitanins (également appelés « tanins hydrolysables ») ;
- les flavonols tels que la quercétine, sous forme glycosylée et/ou aglycone.

Par « anthocyanes », on entend des hétérosides d'anthocyanidols, c'est-à-dire des anthocyanidols portant des sucres. La partie osidique des anthocyanes peut être un monosaccharide (glucose, galactose, rhamnose), un diholoside (rutinose constitué d'un glucose lié à un rhamnose, xyloglucose) ou encore un triholoside. La plupart des anthocyanosides sont des 3-monosides et des 3,5-diosides d'anthocyanidols.

Par « anthocyanidols », on entend une sous-classe de flavonoïdes, dont la structure de base est formée de deux noyaux aromatiques A et B joints par 3 carbones formant avec l'oxygène le cycle C. Les six anthocyanidols les plus courants sont : la cyanidine, la delphinidine, la pélargonidine, la péonidine, la pétunidine et la malvidine.

Dans la baie de canneberge, les sucres représentent environ 45 à 67 % en poids de sucres par rapport au poids total de l'extrait sec. Les sucres présents incluent notamment du glucose, fructose, saccharose et sorbitol.

Enfin, les principaux acides organiques présents dans la canneberge sont l'acide quinique, l'acide citrique, l'acide malique et les acides phénoliques tels que les hydroxybenzoïques et hydroxycinnamiques. La canneberge contient pour 100 g : environ 0,05 g d'acides hydroxybenzoïques (principalement représentés par l'acide benzoïque) et moins de 0,1 g d'acides hydroxycinnamiques représentés majoritairement par les acides p-coumarique, sinapique et caféique.

L'utilisation traditionnelle de la canneberge est essentiellement basée sur des formes transformées de son fruit qui sont :
- le fruit confit,
- le jus, le plus souvent concentré ou séché, et
- les extraits obtenus par purification par divers procédés de différents composés que contient cette plante (dont les proanthocyanidines), soit à partir du fruit, du jus, ou encore des peaux et pulpes regroupées sous l'appellation marc qui sont issues de l'activité de production des jus.

La canneberge présente un pH naturel très bas, proche de 2,3 lorsque le fruit est à maturité. Ce caractère fortement acide rend très délicate l'utilisation du jus de canneberge naturel dans des compositions alimentaires. En effet, de nombreuses personnes rebutent à en boire, à cause de son goût acide et astringent trop prononcé.

C'est pourquoi, le jus de canneberge n'est généralement pas consommé pur ou comme composant majoritaire d'une formulation agro-alimentaire de type jus. En effet, dans les formulations de jus de fruits à base de jus de canneberge qui sont actuellement commercialisées, la teneur massique en jus de canneberge est comprise au maximum entre 15% et 20% par rapport à la masse totale du jus. Elle est généralement de l'ordre de 7%.

BAZINET LAURENT ET AL: "Evolution of cranberry juice physico-chemical parameters during phenolic antioxidant enrichment by electrodialysis with filtration membrane", (2011-11-23) décrit un jus de canneberge désacidifié au cours d'un cycle (cycle 1), au cours duquel son pH atteint à un moment (t) une valeur de 2,9.

SERRE ELODIE ET AL: "Deacidification of cranberry juice by electrodialysis: Impact of membrane types and configurations on acid migration and juice physicochemical characteristics" (2016-02-26) décrit un procédé de désacidification par électrodialyse en présence de différentes membranes (anioniques ou bipolaires) pour atteindre un pH inférieur ou égal à 2.8.

VERA E ET AL: "Comparison of différent methods for deacidification of clarified passion fruit juice", (2003) décrit un procédé de désacidification de jus de fruit de la passion.

US 2010/009049 A1 décrit un procédé consistant à extraire le jus des canneberges à des températures inférieures à 0°C (32°F) et dans lequel le pH n'est pas mentionné.

WO 2010/121203 A1 décrit un procédé dans lequel les flavonoïdes sont séparés des acides et des sucres du jus de canneberge par élution sur des résines adsorbantes, lesdits flavonoïdes adsorbés étant ensuite récupérés et séchés avant d'être utilisés comme complément alimentaire, notamment dans des jus de fruits.

### RÉSUMÉ DE L'INVENTION

Un aspect de l'invention vise un procédé de désacidification d'un jus de canneberge à désacidifier.

Un aspect de l'invention vise un jus de canneberge désacidifié.

Un aspect additionnel de l'invention vise un jus de canneberge désacidifié susceptible d'être obtenu selon le procédé de l'invention.

Un aspect additionnel de l'invention vise une composition alimentaire qui comprend du jus de canneberge désacidifié, par exemple selon le procédé décrit plus bas.

### DESCRIPTION DÉTAILLÉE DE L'INVENTION

L'invention concerne un jus de canneberge désacidifié caractérisé en ce que :
- le pH du jus désacidifié est compris entre 3,2 et 3.8 ;
- le ratio acide quinique /acide malique du jus désacidifié est d'environ 0.85 à environ 0.99 ;
- le jus désacidifié ne contient pas de sucres ajoutés, d'agent de masquage, tels une base ou un complexant, ou de tampon .

Un mode de réalisation consiste en un procédé de désacidification d'un jus de canneberge à désacidifier, caractérisé en ce que :
- le jus de canneberge à désacidifier est élué sur une résine échangeuse d'anions faible pour conduire à un jus de canneberge désacidifié après l'élution;
- la résine échangeuse d'anions faibles est une résine de type acrylique ou de type styrénique ;
- le jus de canneberge à désacidifier est élué sur ladite résine à un taux (BV/h) tel que le jus de canneberge désacidifié après l'élution présente un pH = pKa₁ (acide malique) < pH < pKa (acide benzoïque) ;
où « pKa₁ (acide malique) » représente le pKa de la 1^{ière} acidité de l'acide malique, et « pKa (acide benzoïque) » représente le pKa de l'acide benzoïque .

Par ailleurs, il est à noter qu'une valeur de pH supérieure à environ 6, entraîne une modification substantielle (ou autrement dit une altération), généralement irréversible, de la structure des flavonoïdes.

Dans le cas de la canneberge, parmi ces composés d'intérêt particulièrement sensibles à une valeur trop élevée de pH, on peut citer les anthocyanes dont le changement de structure va être visible du fait que la coloration du jus de canneberge (de nuance naturelle rouge) va virer tout d'abord vers des nuances bleutées si le pH est de l'ordre de 6, puis vers des nuances vertes si le pH se situe entre 8 et 9, et enfin noires si le pH est compris entre 9 et 10.

En d'autres termes, si le pH du jus de canneberge atteint une valeur seuil d'environ 6, cela provoque une dégradation chimique de certains composés d'intérêt que renferme naturellement ce jus. Une fois le composé d'intérêt altéré, il est susceptible de ne plus présenter ses propriétés naturelles qui sont par exemple des propriétés antioxydantes et qui contribuent à conférer au jus canneberge des effets bénéfiques pour la santé (notamment préventifs contre certaines maladies détaillées ci-dessus). C'est pourquoi, il est primordial d'éviter tout stress chimique aux composés d'intérêt tels que notamment les flavonoïdes au cours d'un procédé de désacidification du jus de canneberge, afin d'empêcher leur altération et qu'ils perdent alors leurs propriétés bénéfiques pour la santé.

Un mode de réalisation préféré est donc un procédé dans lequel le pH du jus désacidifié ne dépasse pas (préférablement en aucun temps dudit procédé) la valeur de pH seuil, soit un pH de l'ordre de 6.

Les inventeurs de la présente invention ont mis au point un nouveau procédé de désacidification du jus de canneberge, parfaitement efficace, car il n'altère pas les composés d'intérêt tels que décrits ci-dessus. De plus, le procédé selon l'invention peut être mis en œuvre dans des installations de production et de transformation du jus, et ce sans nécessiter de modifications des dispositifs existants.

Le procédé selon l'invention présente également les avantages de fournir un jus de canneberge, présentant :
- un pH standardisé à la valeur désirée pour l'application souhaitée, cette valeur étant définie comme la consigne à atteindre ;
- une couleur similaire à celle du jus de fruit naturel et donc connue de ses consommateurs;
- dans le cas du jus de canneberge, un indice de concentration pouvant être compris entre 5 et 65 degrés Brix selon qu'une étape ultérieure de concentration sera réalisée sur le jus de canneberge désacidifié ;
- dans le cas du jus de canneberge, un goût astringent fortement réduit par rapport à celui du jus de canneberge naturel ; ce qui le rend très approprié dans les applications alimentaires et médicales, et ce sans nécessiter d'ajouter des sucres et/ou des composés de masquage chimiques, ni de le diluer;
- des propriétés organoleptiques tout à fait similaires à celles du jus de fruit naturel et donc parfaitement reconnaissables par ses consommateurs.

De ce fait, le jus de canneberge désacidifié selon l'invention peut être utilisé dans des formulations de jus de fruits à des teneurs massiques plus élevées que celles actuellement mises en œuvre qui sont d'au plus 15 à 20%, généralement de 7%. Cela présente l'avantage de fournir des formulations de jus de fruits à base de jus de canneberge qui contiennent des teneurs plus élevées en les divers composés d'intérêt que ce jus renferme tels que les vitamines et les flavonoïdes (c'est-à-dire des antioxydants) qui ont été détaillés ci-dessus, et ce par rapport aux jus de fruits à base de jus de canneberge actuellement sur le marché.

Dans un mode de réalisation, le procédé de désacidification est réalisé dans une colonne contenant la résine échangeuse d'anions, ladite colonne présentant une entrée de colonne et une sortie de colonne,
et ladite élution consiste à faire circuler au moins une fois ledit jus de canneberge à désacidifier, de manière à obtenir le jus désacidifié après l'élution.

Un mode de réalisation du procédé consiste à :
- ajuster le débit de circulation dudit jus à désacidifier dans la colonne à un taux égale ou supérieur à 10 BV/heure, comme par exemple entre 10 BV/heure et 250 BV/heure,
- utiliser un volume de jus de canneberge allant de 1 à 20 et préférablement de 5 à 10 BV;
- utilisant une colonne telle que le rapport de la hauteur de la colonne sur le diamètre de la colonne est compris entre 0,3 et 1, plus préférentiellement entre 0,4 et 0,6 ; ou environ 0,5.
de telle sorte que :
- le pH du jus en sortie de colonne ne dépasse pas la valeur de pH seuil à partir de laquelle le au moins un composé d'intérêt est altéré, et préférablement pKa₁ (acide malique) < pH < pKa (acide benzoïque).

Dans un mode de réalisation, le procédé de désacidification est réalisé dans un dispositif de désacidification qui comprend au moins :
- un récipient configuré pour contenir le jus de canneberge à désacidifier qui comprend au moins un composé d'intérêt,
- un récipient de réception du jus désacidifié, et
- une colonne contenant une résine échangeuse d'anions, ladite colonne présentant une entrée de colonne et une sortie de colonne,
ledit procédé comprend au moins une étape qui consiste à faire circuler au moins une fois ledit jus à désacidifier dans ladite colonne de manière à obtenir un jus désacidifié, ledit procédé se caractérise en ce que le débit de circulation dudit jus dans la colonne est compris entre 10 BV/heure et 250 BV/heure et est ajusté de telle sorte que :
- le pH du jus en sortie de colonne ne dépasse pas une valeur de pH seuil à partir de laquelle le au moins un composé d'intérêt est altéré,
- le pH du jus dans le récipient de réception du jus désacidifié augmente jusqu'à une valeur de pH déterminée.

« BV » est l'acronyme anglophone pour « bed volume », c'est-à-dire le volume de résine dans la colonne. En effet, dans le domaine technique de désacidification des jus, qui sont mis en œuvre dans des colonnes remplies de résine échangeuse d'ions, il est parfaitement habituel d'exprimer le débit de circulation (ou autrement dit de passage) du jus dans la colonne en BV/heure. Cela présente l'avantage d'indiquer le débit de façon normalisée, c'est-à-dire quel que soit le volume de la colonne. C'est pourquoi, dans la description qui suit, le débit de circulation du jus de canneberge sera notamment exprimé en BV/heure.

Ainsi, au cours du procédé de désacidification selon l'invention, le débit de circulation du jus de canneberge dans la colonne contenant une résine échangeuse d'anions peut varier tout en restant compris entre 10 BV/heure et 250 BV/heure.

Dans un mode de réalisation du procédé, le volume (BV) du jus de canneberge va de 1 à 20, préférablement de 5 à 10.

Un élément contribuant à l'originalité du procédé selon l'invention réside dans le fait que le jus de canneberge, circule au moins une fois dans une colonne contenant une résine échangeuse d'anions à un débit élevé qui est d'au moins 10 BV/heure et qu'il est également ajusté de telle que sorte que le pH du jus en sortie de colonne ne dépasse pas une valeur seuil de pH à partir de laquelle des composés d'intérêt que renferme naturellement le jus de canneberge, peuvent être altérés.

Cela présente l'avantage que le jus de canneberge, va être désacidifié grâce à la résine d'échangeuse d'anions sans que lesdits composés d'intérêt ne subissent de stress chimique de telle sorte que le jus de canneberge, désacidifié conserve tous ses composés d'intérêt (par exemple les flavonoïdes tels que les anthocyanes) qui sont bénéfiques pour la santé.

Le contenu en proanthocyanides totaux du jus de canneberge désacidifié selon la présente invention est substantiellement le même que le jus de canneberge à désacidifier ayant conduit audit jus de canneberge désacidifié.

Le contenu en proanthocyanides, acides phénoliques et flavonoides du jus de canneberge désacidifié est substantiellement le même que le jus de canneberge à désacidifier ayant conduit audit jus de canneberge désacidifié.

Le contenu en cations inorganique du jus de canneberge désacidifié est substantiellement le même que le jus de canneberge à désacidifier ayant conduit audit jus de canneberge désacidifié. Les cations inorganique sont notamment le potassium, le calcium et le sodium (K, Ca et Na).

Préférablement, le jus traité présente un contenu en proanthocyanides, acides phénoliques, flavonoides ou cations organiques qui n'est pas substantiellement inférieur au contenu du jus de canneberge à désacidifier. Encore plus préférablement, le jus traité présente un contenu de deux, trois ou quatre desdits proanthocyanides, acides phénoliques, flavonoides et cations organiques qui n'est pas substantiellement inférieur au contenu du jus de canneberge à désacidifier.

Ce qu'on entend par « substantiellement» est un contenu ne variant pas à l'extérieur de l'erreur sur la mesure, préférablement ne variant pas à l'extérieur de la plage 95-105%, ou 98-102% et plus préférablement 99-101% du contenu.

Le jus désacidifié selon la présente invention ne contient pas de sucres ajoutés, d'agent de masquage, tels une base ou un complexant, ou de tampon .

L'invention s'étend au jus de canneberge désacidifié selon l'invention, concentré ou non, par exemple ayant un indice de concentration pouvant être compris entre 5 et 65 degrés Brix, et par exemple environ 50 Brix.

Le procédé selon l'invention ne requiert pas de « préconditionement », par exemple par la désactivation partielle de la réactivité de la résine échangeuse d'anions en utilisant un acide (ex. en solution), comme un acide organique alimentaire tel l'acide citrique, l'acide malique, l'acide ascorbique ou une combinaison de ces derniers.

Dans un mode de réalisation de l'invention, le dispositif de désacidification comprend une pluralité de colonnes, par exemple de préférence entre 2 et 10, encore plus préférentiellement entre 3 et 6. Dans un mode de réalisation de l'invention, le dispositif de désacidification comprend trois colonnes.

La mise en œuvre d'une pluralité de colonnes dans des procédés de traitement de jus est parfaitement habituelle et connue de l'homme du métier. Ainsi, cette forme de réalisation du procédé de désacidification selon l'invention est à la portée de l'homme du métier.

Le fait que le dispositif de désacidification comporte plusieurs colonnes permet d'augmenter les performances du procédé de désacidification selon l'invention.

Dans un mode de réalisation du procédé selon l'invention, on fait circuler en boucle ledit jus de canneberge, à désacidifier entre ledit récipient configuré pour contenir le jus de fruit à désacidifier et la colonne. Dans ce mode de réalisation de l'invention, le récipient configuré pour contenir le jus à désacidifier et le récipient de réception du jus désacidifié sont un même et unique récipient. Ainsi, le jus à désacidifier circule en boucle de manière à ce qu'il passe du récipient à l'entrée de colonne, traverse la colonne, puis de la sortie de colonne réintègre le récipient.

Dans un mode de réalisation du procédé selon l'invention, on fait circuler partiellement en boucle ledit jus de canneberge, à désacidifier de telle manière qu'après la sortie de colonne :
- une première partie du jus de canneberge désacidifié réintègre le récipient configuré pour contenir le jus à désacidifier, et qu'
- une deuxième partie du jus de canneberge désacidifié intègre le récipient de réception du jus de canneberge désacidifié.

La mise en œuvre d'une circulation en boucle de manière partielle dans des procédés de traitement de jus de fruit est parfaitement habituelle et connue de l'homme du métier. Cette circulation en boucle de manière partielle est notamment utilisée lorsqu'on souhaite concentrer le jus désacidifié. Ainsi, cette forme de réalisation du procédé de désacidification selon l'invention est à la portée de l'homme du métier.

Dans un autre mode de réalisation du procédé selon l'invention, on fait circuler une seule fois le jus de canneberge, à désacidifier dans ladite colonne.

Ces modes de réalisation du procédé selon l'invention tels que décrits ci-dessus, à savoir mettant en œuvre une pluralité de colonnes, ainsi qu'une circulation par passage unique dans la colonne ou bien une circulation en boucle, le cas échéant une circulation en boucle partielle, peuvent être combinés entre eux fournissant ainsi d'autres modes de réalisation envisageables dans le cadre la présente invention.

Le au moins un composé d'intérêt peut être un des flavonoïdes naturellement présents dans le jus de canneberge qui ont été décrits ci-dessus. Il peut être choisi parmi les anthocyanes, les anthocyanidols, les flavanols, les gallotanins, les ellagitanins et les flavonols. De préférence, le composé d'intérêt est un anthocyane.

L'altération du composé d'intérêt a été expliquée ci-dessus. Il s'agit d'une modification structurelle du composé d'intérêt qui est généralement irréversible. Une fois le composé d'intérêt altéré, il est susceptible de ne plus présenter ses propriétés bénéfiques pour la santé, par exemple ses propriétés antioxydantes.

Dans un mode de réalisation de l'invention, le procédé selon l'invention comprend une étape de prétraitement du jus de canneberge, à désacidifier.

Cette étape de prétraitement peut consister en une clarification du jus par toute technique existante et parfaitement à la portée de l'homme du métier. Parmi ces techniques de clarification, on peut citer la centrifugation et la filtration (notamment la filtration sur membrane, à diatomées ou à plaques).

Par exemple, la clarification est avantageusement mise en œuvre jusqu'à l'obtention d'un jus de canneberge présentant une turbidité inférieure à 500 NTU (« NTU » étant l'acronyme anglophone pour « Nephelometric Turbidity Unit » qui se traduit par « unité de turbidité « Nephelometrique »), de préférence inférieure à 100 NTU, encore plus préférentiellement inférieure à 25 NTU.

Cette étape de prétraitement présente l'avantage d'éviter le colmatage de la colonne.

Au cours du procédé selon l'invention, une résine échangeuse d'anions est utilisée pour capter des acides afin de désacidifier le jus de canneberge. Plus précisément, il se produit dans la colonne un échange d'un anion sur un adsorbant (à savoir la résine qui est un polymère) contre un autre anion.

De manière préférée, la résine est une résine échangeuse d'anions faible. Par exemple, les résines anioniques faibles sont des amines ternaires qui sont neutres à pH supérieur à 10 et ionisés à pH inférieur à 10. Par conséquent, il est entendu que par résine échangeuse faible on entend une résine dont la fonction cationique est dissociée en fonction du pH de la solution.

Les résines échangeuses d'anions faibles présentent l'avantage d'être très spécifiques aux acides faibles et aux acides multivalents. Or, comme cela a été expliqué ci-dessus, le jus de canneberge à désacidifier comprend des acides organiques qui sont des acides faibles. En effet, le pKa (c'est-à-dire la constante d'acidité) de la 1^{ière} acidité de l'acide malique est de 3,4, celui de l'acide quinique de 4,3 et celui de l'acide benzoïque de 4,2.

Dans le cadre de la présente invention, on entend par « acide faible », un acide qui ne se dissocie pas totalement dans l'eau. Un acide est d'autant plus faible que son pKa est élevé.

Dans un mode de réalisation de l'invention, un acide est capturé et un ion hydroxyle est libéré ; ce qui permet au pH d'augmenter et la formation d'une molécule d'eau.

L'équation d'équilibre d'échange (I) est la suivante :

Résine-NH + Acide-H +H₂O=> Résine-NH⁺₂-Acide + OH⁻ (I)

De préférence, la résine échangeuse d'anions est une résine échangeuse de type acrylique ou de type styrénique. De manière avantageuse, il s'agit d'une résine échangeuse d'anions qui est de type acrylique. La résine échangeuse d'anions de type acrylique possède préférablement une capacité entre 1.6-3.2. La résine présente également une vitesse d'échange initiale égale ou supérieure à +0,10 unité de pH/minute observée après 5 minutes de contact du jus à désacidifier avec la résine dans un ratio volumique de 5 :1 (jus :résine). La taille de particule de la résine varie entre 300-600uM.

Un exemple de résine échangeuse d'anions faibles de type acrylique est le modèle CR5550 commercialisé par la société DOW CHEMICAL sous la dénomination commerciale AMBERLITE®.

De manière avantageuse, le rapport de la hauteur de la colonne sur le diamètre de la colonne est compris entre 0,3 et 1, plus préférentiellement entre 0,4 et 0,6. De manière tout à fait préférée, ce rapport est de 0,5.

Lorsque ledit rapport est compris entre 0,3 et 1, cela présente l'avantage de pouvoir faire circuler le jus de canneberge, à désacidifier dans la colonne à des débits très élevés qui sont supérieurs à ceux habituellement mis en œuvre au cours des procédés de désacidification du jus dans des colonnes remplis de résine échangeuse d'anions, et ce à des pressions comprises entre 2 bars et 5 bars.

Le volume total de la colonne peut représenter entre 1 et 2, de préférence entre 1,3 et 1,7, encore plus préférentiellement entre 1,4 et 1,6 fois, le volume de la résine qu'elle contient.

Préférentiellement, la circulation du jus de canneberge, à désacidifier est réalisée dans la colonne en mode ascendant (en anglais « up flow »). Dans le domaine des résines d'échanges d'ions, il est tout à fait classique de mettre en œuvre une circulation du jus à traiter en mode ascendant, lorsque la résine capture des espèces chimiques (dans le cas présent des acides faibles). En effet, lorsque la résine capture des espèces, elle augmente de volume et le fait que la circulation du jus soit en mode ascendant évite des phénomènes d'augmentation de pression qui pourraient bloquer cette expansion de la résine.

La hauteur du lit de la résine échangeuse d'anions peut être comprise entre 30 cm et 200 cm, de préférence entre 50 cm et 100 cm.

Dans un mode réalisation du procédé, le pH du jus de jus de canneberge, en sortie de colonne ne dépasse jamais la valeur de pH seuil majorée de 0,5 unité de pH.

De manière préférée, dans le cas du jus de canneberge, la valeur de pH seuil à partir de laquelle le au moins un composé d'intérêt est altéré est comprise entre 6 et 9.

Dans des modes de réalisation du procédé selon l'invention, le pH du jus de canneberge, en sortie de colonne est supérieur au pKa d'au moins un acide faible déterminé que contient naturellement le jus de canneberge. Ces modes de réalisation permettent de capturer plus sélectivement au moins un acide faible déterminé parmi les acides faibles que renferme naturellement le jus de canneberge. Si le pH du jus de canneberge, circulant dans la colonne est inférieur au pKa d'un acide faible déterminé que contient naturellement ce jus, alors cet acide est très peu capturé par la résine échangeuse d'anions.

La capture sélective d'au moins un acide faible au cours du procédé de désacidification selon l'invention est particulièrement avantageuse. En effet, il est nécessaire de désacidifier le jus de canneberge, pour le rendre consommable en l'absence de dilutions et/ou d'ajouts d'autres composés, mais certains acides qu'il renferme ont également des propriétés bénéfiques pour la santé et/ou pour le goût du jus. C'est pourquoi, il est intéressant de ne pas capturer ces acides.

Dans le jus de canneberge, l'acide malique est très concentré et confère à ce jus un goût trop acide et astringent. C'est pourquoi, on cherche à le capturer avec la résine échangeuse d'anions au cours du procédé selon l'invention, afin d'en réduire sa concentration. Cependant, les acides quinique et benzoïque également présents dans le jus de canneberge naturel présentent l'avantage d'être des conservateurs naturels. Il est utile de les conserver majoritairement dans le jus de canneberge et donc d'éviter leur capture par la résine échangeuse d'anions au cours du procédé selon l'invention.

Au vu des valeurs de pKa des acides malique, quinique et benzoïque rappelées ci-dessus, si l'on souhaite capturer sélectivement l'acide malique au cours du procédé de désacidification selon l'invention, il faut que la valeur de pH du jus de canneberge en sortie de colonne soit supérieure au pKa de l'acide malique mais inférieure aux pKa de l'acide quinique et benzoïque.

Dans un mode de réalisation de l'invention, la valeur de pH du jus de canneberge en sortie de colonne est comprise entre le pKa de la 1^{ière} acidité de l'acide malique (c'est-à-dire environ 3,4) et le pKa de l'acide benzoïque (c'est-à-dire environ 4,2). Dans ce mode de réalisation, les acides benzoïque et quinique sont peu capturés par la résine échangeuse d'anions. Leur concentration est donc maintenue quasiment constante dans le jus de canneberge, et ce malgré la désacidification dudit jus au cours du procédé selon l'invention.

Ainsi, une valeur de pH du jus de canneberge en sortie de colonne d'environ 3,5 est particulièrement appropriée pour capturer plus sélectivement l'acide malique au cours du procédé de désacidification selon l'invention, et donc maintenir dans le jus désacidifié les concentrations en acides quinique et benzoïque substantiellement égales à celles du jus de canneberge avant sa désacidification.

Le jus de canneberge en sortie de colonne présente un ratio acide quinique /acide malique allant de 0.85 à 0.99.

Bien entendu, dans ces modes de réalisation de l'invention dans lesquels on capture en outre de manière sélective un ou plusieurs acides faibles, le jus de canneberge, désacidifié conserve ses propriétés organoleptiques et ses vertus thérapeutiques, car la valeur de pH du jus de canneberge en sortie de colonne est également inférieure à la valeur de pH seuil à partir de laquelle des composés d'intérêt tels que les flavonoïdes peuvent être altérés.

Selon l'invention, la valeur de pH déterminée pour le jus de canneberge st comprise entre 3,2 et 3,8.

De manière tout à fait préférée, dans le cas du jus de canneberge, la valeur de pH déterminée est environ 3,5. En d'autres termes, dans ce mode de réalisation du procédé selon l'invention, on fait circuler dans la colonne le jus de canneberge jusqu'à ce que le pH du jus de canneberge qui était initialement compris entre 2,3 et 2,5 (c'est-à-dire avant sa désacidification) atteigne la valeur d'environ 3,5 à l'issue du procédé de désacidification.

La circulation du jus de de canneberge, dans la colonne peut être réalisée au moyen d'une pompe dont est équipé le dispositif de désacidification. De manière préférée, la pompe est une pompe volumétrique.

Le dispositif de désacidification peut en outre comprendre un pH-mètre pour mesurer le pH du jus de canneberge, désacidifié en sortie de la colonne.

Dans un mode de réalisation de l'invention, le débit de circulation du jus de canneberge, dans la colonne est ajusté au moyen d'un régulateur PID (« PID » étant l'acronyme pour « Proportionnel, Intégrateur, Dérivateur ») dont est équipé le dispositif de désacidification. Un régulateur PID est un dispositif classiquement utilisé dans l'industrie qui permet d'effectuer un asservissement en boucle fermée d'un procédé.

Ainsi, dans un mode de réalisation de l'invention, le dispositif de désacidification comprend en outre une pompe et un pH-mètre pour mesurer le pH du jus désacidifié en sortie de la colonne et, à partir des valeurs de pH du jus de canneberge, en sortie de colonne qui sont mesurées par ledit pH-mètre et que reçoit le régulateur PID, ledit régulateur PID, à partir d'un algorithme de calcul, délivre un signal de commande de débit à la pompe de manière à ce que le débit de circulation dudit jus de canneberge dans la colonne est compris entre 10 BV/heure et 250 BV/heure et est ajusté de telle sorte que :
- le pH du jus de canneberge en sortie de colonne ne dépasse pas une valeur de pH seuil à partir de laquelle le au moins un composé d'intérêt est altéré,
- le pH du jus de canneberge dans le récipient de réception du jus de canneberge désacidifié augmente jusqu'à une valeur de pH déterminée.

Dans un mode de réalisation de l'invention, le dispositif comprend en outre un pH-mètre pour mesurer le pH dans le récipient contenant le jus de canneberge, désacidifié. Ainsi, on peut suivre l'évolution du pH du jus désacidifié, et notamment surveiller qu'il a atteint la valeur de pH déterminée.

Dans les modes de réalisation de l'invention dans lesquels la résine capture sélectivement au moins un acide faible déterminé, le débit de circulation du jus de canneberge est en outre ajusté de telle sorte que le pH du jus de canneberge, en sortie de colonne est supérieur au pKa dudit au moins un acide faible déterminé.

Le réglage du régulateur PID, c'est-à-dire notamment la mise au point d'un algorithme de calcul pour la délivrance d'un signal de commande de débit à la pompe est parfaitement à la portée de l'homme du métier. En effet, l'homme du métier saura, à partir d'essais, de routine régler le régulateur PID pour ajuster le débit de circulation dans la colonne du jus de canneberge, au cours du procédé selon l'invention.

La vitesse d'échange dans la résine échangeuse d'anions dépend de la vitesse de diffusion des anions à l'intérieur de la résine (le degré de réticulation étant un des facteurs de cette vitesse de diffusion) et de la vitesse de diffusion à l'interface résine-jus de canneberge (diffusion à travers le film liquide autour de chaque grain de résine). De manière générale, la vitesse d'échange augmente avec la finesse des particules de résine, la mobilité des anions échangés, la concentration et la température du jus considéré. En augmentant la vitesse de circulation du jus de canneberge, à travers le lit de la résine, on limite le temps de contact résine-jus de canneberge. On limite donc l'échange ; ce qui permet d'ajuster le pH en sortie de la colonne.

Si le débit de circulation de jus de de canneberge, est supérieur à la vitesse d'équilibre d'échange de l'équation (I) détaillée ci-dessus, alors la réaction d'échange est incomplète, la résine n'a pas le temps de capturer tous les acides présents dans le jus de canneberge.

Plus le débit de circulation augmente, plus le pH en sortie de colonne va avoir tendance à diminuer. A contrario, plus le débit de circulation diminue, plus le pH du jus de canneberge, en sortie de colonne va avoir tendance à augmenter.

Ainsi, en régulant le débit de circulation du jus de canneberge, dans la colonne, on peut ajuster le pH du jus en sortie de colonne.

Au début du procédé selon l'invention, la résine échangeuse d'anions comporte de nombreux sites actifs aptes à capturer les acides; ce qui conduit à une variation importante du pH du jus de canneberge entre l'entrée et la sortie de colonne. Afin d'éviter un pH du jus de canneberge, en sortie de colonne trop élevé (c'est-à-dire supérieur à la valeur de pH seuil à partir de laquelle les composés d'intérêt sont altérés), il est nécessaire que le débit de circulation du jus de canneberge, dans la colonne soit élevé au début du procédé de manière à ce que tous les acides du jus de canneberge, n'aient pas le temps d'être capturés par les sites actifs de la résine.

Par exemple, dans le cas du jus de canneberge, la cinétique telle qu'appliquée permet de fixer préférentiellement l'acide malique, et capte ainsi une proportion de l'acide malique, avec une forte sélectivité.

Ensuite, au fur et à mesure que les sites actifs de la résine sont saturés, le débit de circulation du jus de de canneberge, dans la colonne peut être diminué. Ainsi, l'ajustement du débit de circulation du jus de canneberge, dans la colonne au cours du procédé est essentiel dans le cadre de la présente invention.

Par exemple, au début du procédé selon l'invention, c'est-à-dire pendant les environ 2 à 4 premières minutes de la circulation du jus de canneberge, dans la colonne, le débit est de préférence compris entre 100 BV/heure et 150 BV/heure. Ce débit de circulation est beaucoup plus élevé que les débits de circulation classiquement mis en œuvre dans une colonne d'échanges d'ions pour la désacidification de jus qui sont généralement de l'ordre de 5 BV/heure.

Afin que le pH du jus en sortie de colonne ne franchisse pas la valeur seuil à partir de laquelle des composés d'intérêt tels que les flavonoïdes sont altérés, et le cas échéant qu'il soit supérieur au pKa d'au moins un acide faible déterminé que contient naturellement le jus de canneberge, il est nécessaire d'imposer un débit de circulation du jus, très élevé, de plus de 100 BV/heure en tout début de procédé. Ensuite, ce débit de circulation peut être diminué et maintenu constant de telle sorte que le pH du jus de canneberge, désacidifié augmente progressivement jusqu'à la valeur de pH déterminée qui est pour le cas de la canneberge de préférence de l'ordre de 3,5.

A l'issue du au moins un passage dans la colonne du jus à désacidifier, le procédé de désacidification selon l'invention peut en outre comprendre une étape de concentration du jus de canneberge désacidifié. Par exemple, il peut s'agir d'une technique telle que l'osmose inverse ou bien l'évaporation.

Cette étape supplémentaire de concentration du jus de canneberge désacidifié est parfaitement à la portée de l'homme du métier.

Par ailleurs, afin de récupérer les acides qui ont été capturés par la résine échangeuse d'anions lors du passage du jus de canneberge, il est possible de réaliser à l'issue du procédé de désacidification selon l'invention, une étape de saturation de la résine. En effet, les acides captés par la résine peuvent présenter un intérêt dans d'autres applications que le jus. Par exemple, dans le cas du jus de canneberge, cette régénération va permettre de valoriser l'acide malique ainsi capturé au cours du procédé selon l'invention.

L'étape de saturation est parfaitement à la portée de l'homme du métier. Par exemple, elle peut être mise en œuvre avec une solution d'acide chlorhydrique, de préférence à une concentration de 1 mol/L (73 g/L de résine).

De plus, une étape de régénération de la résine échangeuse d'anions peut être réalisée afin de pouvoir effectuer à nouveau avec cette même résine une désacidification d'un jus de canneberge.

Cette étape de régénération de la résine échangeuse d'anions est également parfaitement à la portée de l'homme du métier. Par exemple, elle peut être réalisée avec une solution de soude à 1 mol/L (80 g/L de résine).

A l'issue du procédé de désacidification d'un jus de canneberge, on peut procéder ainsi :
- on effectue une étape de saturation de la résine, par exemple telle que décrite ci-dessus ;
- Optionnellement suivie d'un rinçage à l'eau, de préférence un rinçage rapide ;
- puis une étape de régénération, par exemple telle que décrite ci-dessus ;
- optionnellement suivie d'un ou plusieurs rinçages à l'eau (par exemple un rinçage lent, puis un rinçage rapide).

La présente invention a aussi pour objet une composition alimentaire qui comprend du jus de canneberge, par exemple désacidifié selon le procédé selon l'invention, tel que décrit ci-dessus.

Dans les compositions alimentaires, le jus de canneberge, concentré ou non (par exemple ayant un indice de concentration pouvant être compris entre 5 et 65 degrés Brix, et par exemple environ 50 Brix) présente l'avantage de pouvoir se substituer à des produits élaborés avec des apports en sucres exogènes tels que saccharose ou autre tout autre sucres simple ou complexe qui sont néfastes pour la santé.

La composition alimentaire peut être une boisson, gazeuse ou non. Par exemple, il peut s'agir d'un jus de fruit.

Outre les boissons, la composition alimentaire peut être également choisie parmi les sorbets, les glaces, les produits laitiers et les sauces.

Des exemples de compositions alimentaires selon l'invention sont :
- des thés glacés, notamment des thés glacés comprenant un mélange de canneberge avec de la framboise, du citron vert ou de la mangue ;
- des boissons gazeuses comprenant un mélange de canneberge avec du citron, du pamplemousse ou du guarana ;
- des sirops concentrés non-alcoolisés comprenant un mélange de canneberge avec du pamplemousse rose, de l'orange sanguine, de la pêche de vigne ou de la grenadine ;
- des sirops pour cafés et infusions à base de canneberge ;
- des boissons alcoolisées, par exemple des liqueurs (notamment des liqueurs dont le degré alcoolique est 25) ;
- des boissons de cocktails à base de canneberge et de mojito ou de vodka ;
- -des produits laitiers ;
- des vinaigrettes, par exemple des vinaigrettes à base de canneberge et de framboise ou de vinaigre balsamique ;
- des sauces salées, par exemple des sauces salées comprenant un mélange de canneberge et poivre, pesto, oignon frit, échalote ou encore de lime ;
- des sauces de type barbecue à base de canneberge et éventuellement de piments ;
- des garnitures à desserts, par exemple des garnitures à desserts comprenant un mélange de canneberge avec des fruits rouges, de la stracciatella, du chocolat, du sucre de canne, de la noix de coco ou encore du miel.

Ainsi, la composition alimentaire peut être choisie parmi les boissons, les sorbets, les glaces, les sauces, les garnitures à desserts et les vinaigrettes.

La présente invention a aussi pour objet une composition nutraceutique qui comprend du jus de canneberge désacidifié selon l'invention tel que décrit ci-dessus.

La présente invention a aussi pour objet une composition qui comprend du jus de canneberge désacidifié tel que décrit ci-dessus pour son utilisation dans la prévention des infections urinaires.

L'invention sera mieux comprise à l'aide de la description détaillée qui est exposée ci-dessous en référence au dessin annexé représentant, à titre d'exemple non limitatif, des formes de réalisation de dispositifs de désacidification dans lesquels peuvent être mis en œuvre le procédé selon l'invention, ainsi que des données expérimentales relatives audit procédé.

### DESCRIPTION DES FIGURES :

La figure 1 représente de manière schématique un dispositif dans lequel peut être réalisé le procédé de désacidification selon l'invention selon un 1^{ier} mode de réalisation.
La figure 2a représente de manière schématique un dispositif dans lequel peut être réalisé le procédé de désacidification selon l'invention selon un 2^{ième} mode de réalisation.
La figure 2b représente de manière schématique un dispositif dans lequel peut être réalisé le procédé de désacidification selon l'invention selon un 3^{ième} mode de réalisation.
La figure 2c représente de manière schématique un dispositif dans lequel peut être réalisé le procédé de désacidification selon l'invention selon un 4^{ième} mode de réalisation.
La figure 3 est un graphique de l'évolution des valeurs de pH en fonction du BV au cours des expérimentations de désacidification par passage unique de jus de canneberge dans une colonne d'échanges d'anions remplie d'une 1^{ière} résine à un débit de 5 BV/heure.
La figure 4 est un graphique de l'évolution des valeurs de pH en fonction du BV au cours des expérimentations de désacidification par passage unique de jus de canneberge dans une colonne d'échanges d'anions remplie d'une 2^{ième} résine à un débit de 5 BV/heure.
La figure 5 est un graphique représentant l'évolution des valeurs de pH, et du débit de circulation en fonction du temps pour des expérimentations de désacidification mises en œuvre avec une 1^{ière} résine et avec une recirculation à une charge de 8 BV.
La figure 6 est un graphique représentant l'évolution des valeurs de pH, et du débit de circulation en fonction du temps pour des expérimentations de désacidification mises en œuvre avec une 1^{ière} résine et avec une recirculation à une charge de 10 BV.
La figure 7 est un graphique représentant l'évolution des valeurs de pH, et du débit de circulation en fonction du temps pour des expérimentations de désacidification mises en œuvre avec une 1^{ière} résine et avec une recirculation à une charge de 9 BV.
La figure 8 est un graphique représentant l'évolution des valeurs de pH, et du débit de circulation en fonction du temps pour des expérimentations de désacidification mises en œuvre avec une 1^{ière} résine et avec une recirculation à une charge de 9 BV.
La figure 9 est un graphique représentant l'évolution des valeurs de pH, et du débit de circulation en fonction du temps pour des expérimentations de désacidification mises en œuvre avec une 1^{ière} résine et avec une recirculation à une charge de 8 BV.
La figure 10 est un graphique représentant l'évolution des valeurs de pH, et du débit de circulation en fonction du temps pour des expérimentations de désacidification mises en œuvre avec une 2^{ième} résine et avec une recirculation à une charge de 6 BV.
La figure 11 est un graphique représentant l'évolution des valeurs de pH, et du débit de circulation en fonction du temps pour des expérimentations de désacidification mises en œuvre avec une 2^{ième} résine et avec une recirculation à une charge de 8 BV.
La figure 12 est un graphique représentant l'évolution des valeurs de pH, et du débit de circulation en fonction du temps pour des expérimentations de désacidification mises en œuvre avec une 2^{ième} résine et avec une recirculation à une charge de 10 BV.
La figure 13 est un graphique représentant la variation de la durée de l'expérimentation, du pH à l'issue de l'expérimentation en fonction de la charge de jus de canneberge à désacidifier pour les expérimentations réalisées avec la 1^{ière} résine.
La figure 14 est un graphique représentant la variation de la durée de l'expérimentation, du pH à l'issue de l'expérimentation en fonction de la charge de jus de canneberge à désacidifier pour les expérimentations réalisées avec la 2^{ième} résine.
La figure 15 est un graphique de l'évolution de la concentration en chlorures, acide malique et acide quinique en fonction du BV cumulé d'acide chlorhydrique, puis d'eau.
La figure 16 est un graphique de l'évolution de la concentration en chlorures, acide malique et acide quinique en fonction du BV cumulé d'acide chlorhydrique, puis d'eau.

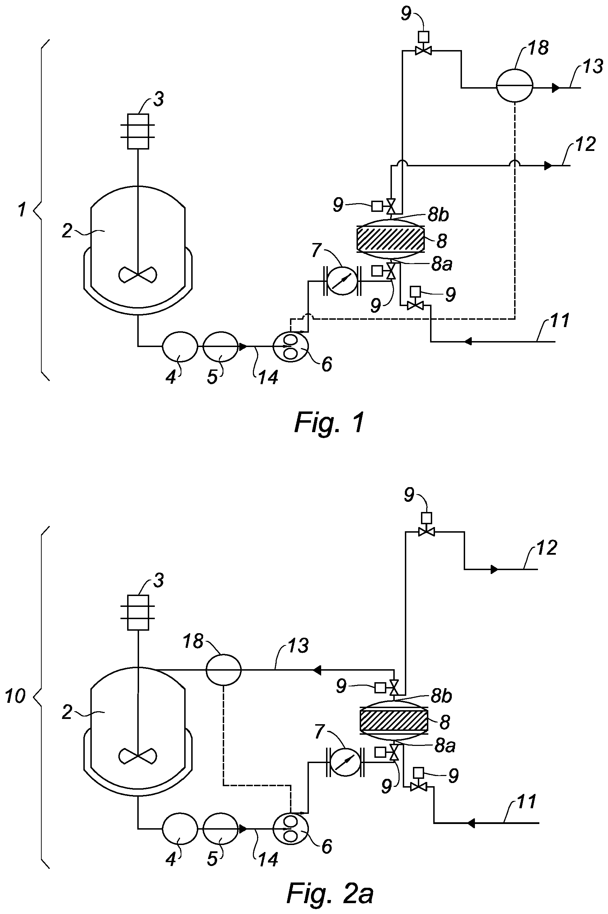

La figure 1 représente de manière schématique un dispositif 1 dans lequel peut être réalisé le procédé de désacidification selon l'invention selon un 1^{ier} mode de réalisation dans lequel le jus de canneberge à désacidifier circule une seule fois dans une colonne d'échanges d'anions.
La figure 2a représente de manière schématique un dispositif 10 dans lequel peut être réalisé le procédé de désacidification selon l'invention selon un 2^{ième} mode de réalisation dans lequel le jus de canneberge à désacidifier circule en boucle dans une colonne d'échanges d'anions.

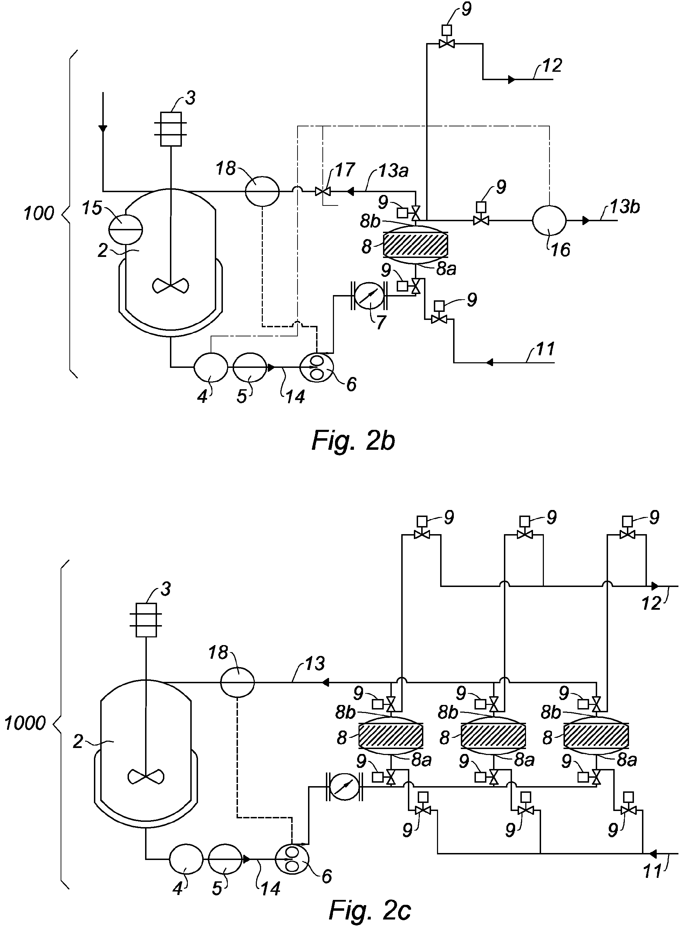

La figure 2b représente de manière schématique un dispositif 100 dans lequel peut être réalisé le procédé de désacidification selon l'invention selon un 3^{ième} mode de réalisation dans lequel le jus de canneberge à désacidifier circule en boucle partiellement dans une colonne d'échanges d'anions.
La figure 2c représente de manière schématique un dispositif 1000 équipé de trois colonnes d'échanges d'anions et dans lequel peut être réalisé le procédé de désacidification selon l'invention selon un 4^{ième} mode de réalisation dans lequel le jus de canneberge à désacidifier circule en boucle dans les trois colonnes d'échanges d'anions.

Les éléments structurels communs aux dispositifs 1, 10, 100 et 1000 et qui ont la même fonction portent la même référence numérique quelle que soit la figure 1, 2a, 2b et 2c.

Les dispositifs 1, 10, 100 et 1000 comprennent :
- une cuve 2 configurée pour contenir le jus de canneberge à désacidifier - autrement dit un bac d'alimentation pour contenir le jus brut à désacidifier ;
- un moteur 3 pour agiter le contenu de la cuve 2 ;
- une colonne d'échange d'anions 8 (le dispositif 1000 comprend trois colonnes 8) présentant une entrée de colonne 8a et une sortie de colonne 8b ;
- un débitmètre 4 de régulation du jus;
- une sonde de température 5 ;
- une pompe 6 ;
- un indicateur de pression 7 ;
- des vannes 9 ;
- un pH-mètre 18 pour mesurer le pH du jus en sortie de colonne 8b ;
- un canal 11 d'amenée d'une solution de régénération de la colonne 8 ;
- un canal 12 d'évacuation de la solution de régénération après son passage dans la colonne 8 ;
- un canal 14 d'amenée du jus à désacidifier à la colonne 8 ;

La solution de régénération est utilisée au cours d'une étape de régénération de la résine échangeuse d'anions que contient la colonne 8. Cette étape de régénération a été mentionnée ci-dessus.

Sur le canal 14 sont donc disposés le débitmètre 4, la sonde de température 5 et la pompe 6.

Les pointillés sur les figures 1, 2a, 2b et 2c schématisent un régulateur PID. A partir des valeurs de pH en sortie de colonne 8b qui sont mesurées par le pH-mètre 18 et que reçoit le régulateur PID, ledit régulateur PID, à partir d'un algorithme de calcul, délivre un signal de commande de débit à la pompe 6 de manière à ce que le débit de circulation dudit jus dans la colonne 8 est compris entre 10 BV/heure et 250 BV/heure et est ajusté comme cela a été détaillé ci-dessus.

Les dispositifs 1, 10 et 1000 comprennent en outre un canal d'évacuation 13 du jus désacidifié de la colonne 8.

Dans le cas du dispositif 1, ledit canal 13 est relié à un récipient de réception de jus désacidifié non représenté sur la figure 1 de manière à collecter le jus ayant circulé dans la colonne 8 et qui a donc été désacidifié.

Dans le cas du dispositif 10, le jus à désacidifier circule en boucle entre la cuve 2 et la colonne 8 lorsque le procédé de désacidification selon l'invention est mis en œuvre. La cuve 2 est donc également le récipient de réception du jus désacidifié. Le canal 13 est relié à la cuve 2 de manière à ce que le jus ayant circulé dans la colonne 8 soit réintégré dans la cuve 2.

Il en est de même pour le dispositif 1000 qui comprend trois colonnes 8. Le canal 13 est relié à la cuve 2 de manière à ce que le jus ayant circulé dans les trois colonnes 8 soit réintégré dans la cuve 2.

Le dispositif 100 est configuré pour que le jus circule en boucle partiellement entre la cuve 2 et la colonne 8.

Le dispositif 100 comprend en outre un moyen de mesure de niveau 15, un débitmètre 16, une vanne proportionnelle 17, un premier canal d'évacuation 13a d'une première partie du jus désacidifié de la colonne 8 vers la cuve 2 et un deuxième canal 13b d'évacuation 13b d'une deuxième partie du jus désacidifié de la colonne 8 vers un récipient de réception de cette deuxième partie du jus qui n'est pas représenté sur la figure 2b.

Le réglage de la vanne proportionnelle 17 permet la répartition souhaitée du jus désacidifié entre sa réintégration dans la cuve 2 et son évacuation vers un récipient de réception du jus désacidifié. Comme expliqué ci-dessus, la deuxième partie du jus désacidifié qui est donc évacuée par le canal 13b pourra être soumis à un traitement de concentration parfaitement à la portée de l'homme du métier.

Sur la figure 2b, les pointillés reliant la vanne proportionnelle 17 au débitmètre 4 et au débitmètre 16 schématisent la boucle de régulation du régulateur PID permettant l'ajustement de l'ouverture de la vanne proportionnelle 17 pour piloter le débit de recyclage (c'est-à-dire le débit de jus de canneberge désacidifié qui est réintégré dans la cuve 2) en fonction du différentiel entre le débit du jus de canneberge en entrée de colonne 8a et le débit de collecte du jus désacidifié mesuré par le débitmètre 16.

### PARTIE EXPERIMENTALE

Les expérimentations qui sont détaillées ci-dessous concernent la mise en œuvre de la désacidification d'un jus de canneberge qui présentait initialement les caractéristiques suivantes :
- un indice de concentration de 7,6 degrés Brix ;
- un pH de 2,47 ;
- une couleur rouge ;
- une acidité totale de 0,098 équivalent/L ;
- une concentration en acide malique de 9,47 g/L ;
- une concentration en acide quinique de 6,63 g/L.

La matière sèche du jus de canneberge a été estimée en mesurant l'indice Brix avec une échelle saccharose.

Le dosage des acides a été réalisé par chromatographie en phase liquide à haute performance (ci-après abrégée « HPLC » qui est l'acronyme anglophone pour « High Performance Liquide Chromatography ») avec :
- une colonne commercialisée par la société BIO-RAD sous la dénomination commerciale Aminex HPX-87H de dimensions 7,8 x 300 ;
- un éluant qui était une solution d'acide sulfurique à 3 mmol/L mis en œuvre avec un débit d'élution de 1 mL/minute à 60°C
- l'analyse des cations est effectué par ICP (Inductively Coupled Plasma -torche à plasma d'azote), sur un appareil de marque Agilent.

Les expérimentations se décomposent en les trois parties suivantes :
a) la désacidification du jus de canneberge en mode stationnaire (ou autrement dit en mode « Batch ») dans un bécher contenant une résine échangeuse d'anions ;
b) la désacidification du jus de canneberge par passage unique dans une colonne remplie d'une résine échangeuse d'anions ;
c) la désacidification du jus de canneberge par circulation en boucle dans une colonne remplie d'une résine échangeuse d'anions.

Toutes les expérimentations détaillées ci-dessous ont été réalisées à 20°C.

Au cours de toutes ces expérimentations de désacidification, la valeur de pH déterminée du jus de canneberge à atteindre a été fixée à 3,5. Autrement dit, 3,5 était la « valeur cible » à atteindre du pH du jus de canneberge à l'issue de toutes ces expérimentations de désacidification.

Les 5 résines échangeuses d'anions qui ont été utilisées au cours de ces expérimentations présentaient les caractéristiques qui sont détaillées dans le tableau 1 ci-dessous, à savoir :
- le modèle,
- la société commercialisant la résine et sous quelle dénomination commerciale,
- la structure,
- la capacité totale théorique indiquée par son fournisseur et exprimée en équivalent/L,
- la granulométrie des billes de la résine (exprimée en µm),
- le coefficient d'uniformité (abrégé ci-après « CU »), où CU = d60/dl0 (diamètre pour 60% de la masse des billes/ diamètre pour 10%de la masse des billes).

**Tableau 1 détaillant les caractéristiques des 5 résines utilisées au cours des expérimentations**

| | Résine 1 | Résine 2 | Résine 3 | Résine 4 | Résine 5 |
|---|---|---|---|---|---|
| Modèle | S5221 | CR5550 | A365 | Dowex66 | FPA51 |
| Société | LANXESS | DOW CHEMICAL | RHOM & HAAS | DOW CHEMICAL | DOW CHEMICAL |
| Dénomination commerciale | LEWATIT® | AMBERLITE® | DUOLITE® | AMBERLITE® | AMBERLITE ® |
| Structure | acrylique gel | acrylique gel | acrylique gel | Polystyrène macroréticulée | Polystyrène macroréticul ée |
| Capacité (équivalent/L) | 3.0-3.2 | 1,6 | 3,5 | 1,3 | 1,3 |
| Taille (µm) | 550 | 450 | 550 | 550 | 550 |
| CU | 1,8 | 1,2 | 1,8 | 1,1 | 1,8 |

### A - DESACIDIFICATION DU JUS DE CANNEBERGE EN MODE STATIONNAIRE :

Deux essais (El et E2) ont été effectués pour chacune des résines 1 à 5 selon le protocole expérimental suivant :
Dans un bêcher, on a mis en contact 50 mL du jus de canneberge avec 10 mL de la résine choisie de manière à obtenir un mélange.

Le mélange a été agité en continu avec un agitateur aimanté et le pH du surnageant a été mesuré régulièrement.

Une fois l'équilibre atteint, on a déterminé la vitesse d'échange initiale à partir de la variation de pH observée au bout de 5 minutes.

Le tableau 2 ci-dessous détaille en fonction du temps (exprimé en minutes) le pH mesuré dans le mélange contenant la résine 1 au cours du 1^{ier} essai (El) et du 2^{ième} essai (E2), ainsi que la valeur moyenne du pH calculé (pH moyen).

**Tableau 2 détaillant les valeurs de pH mesurées dans le mélange contenant la résine 1**

| Temps (minutes) | 0 | 0,5 | 1 | 2 | 3 | 5 | 10 | 20 |
|---|---|---|---|---|---|---|---|---|
| pH E1 | 2,53 | 2,69 | 2,77 | 2,90 | 2,99 | 3,16 | 3,49 | 4,09 |
| pH E2 | 2,53 | 2,65 | 2,74 | 2,86 | 2,96 | 3,12 | 3,45 | 4,00 |
| pH moyen | 2,53 | 2,67 | 2,76 | 2,88 | 2,98 | 3,14 | 3,47 | 4,05 |

Pour la résine 1, une vitesse d'échange initiale de 0,12 unité de pH/minute a été déterminée.

Le tableau 3 ci-dessous détaille en fonction du temps (exprimé en minutes) le pH mesuré dans le mélange contenant la résine 2 au cours du 1^{ier} essai (El) et du 2^{ième} essai (E2), ainsi que la valeur moyenne du pH calculée (pH moyen).

**Tableau 3 détaillant les valeurs de pH mesurées dans le mélange contenant la résine 2**

| Temps (minutes) | 0 | 0,5 | 1 | 2 | 3 | 5 | 10 | 20 |
|---|---|---|---|---|---|---|---|---|
| pH E1 | 2,53 | 2,87 | 2,99 | 3,17 | 3,32 | 3,53 | 3,84 | 4,02 |
| pH E2 | 2,53 | 2,83 | 2,96 | 3,11 | 3,24 | 3,46 | 3,76 | 3,88 |
| pH moyen | 2,53 | 2,85 | 2,98 | 3,14 | 3,28 | 3,50 | 3,80 | 3,95 |

Pour la résine 2, une vitesse d'échange initiale de 0,19 unité de pH/minute a été déterminée.

Le tableau 4 ci-dessous détaille en fonction du temps (exprimé en minutes) le pH mesuré dans le mélange contenant la résine 3 au cours du 1^{ier} essai (El) et du 2^{ième} essai (E2), ainsi que la valeur moyenne du pH calculée (pH moyen).

**Tableau 4 détaillant les valeurs de pH mesurées dans le mélange contenant la résine 3**

| Temps (minutes) | 0 | 0,5 | 1 | 2 | 3 | 5 | 10 | 20 |
|---|---|---|---|---|---|---|---|---|
| pH E1 | 2,53 | 2,64 | 2,71 | 2,75 | 2,81 | 2,9 | 3,16 | 3,53 |
| pH E2 | 2,53 | 2,65 | 2,69 | 2,74 | 2,79 | 2,84 | 3,09 | 3,48 |
| pH moyen | 2,53 | 2,65 | 2,70 | 2,75 | 2,80 | 2,87 | 3,13 | 3,51 |

Pour la résine 3, une vitesse d'échange initiale de 0,07 unité de pH/minute a été déterminée.

Le tableau 5 ci-dessous détaille en fonction du temps (exprimé en minutes) le pH mesuré dans le mélange contenant la résine 4 au cours du 1^{ier} essai (El) et du 2^{ième} essai (E2), ainsi que la valeur moyenne du pH calculée (pH moyen).

**Tableau 5 détaillant les valeurs de pH mesurées dans le mélange contenant la résine 4**

| Temps (minutes) | 0 | 0,5 | 1 | 2 | 3 | 5 | 10 | 20 |
|---|---|---|---|---|---|---|---|---|
| pH E1 | 2,53 | 2,64 | 2,66 | 2,72 | 2,76 | 2,84 | 3,00 | 3,22 |
| pH E2 | 2,53 | 2,64 | 2,66 | 2,71 | 2,76 | 2,84 | 2,99 | 3,22 |
| pH moyen | 2,53 | 2,64 | 2,66 | 2,72 | 2,76 | 2,84 | 3,00 | 3,22 |

Pour la résine 4, une vitesse d'échange initiale de 0,06 unité de pH/minute a été déterminée.

Le tableau 6 ci-dessous détaille en fonction du temps (exprimé en minutes) le pH mesuré dans le mélange contenant la résine 5 au cours du 1^{ier} essai (El) et du 2^{ième} essai (E2), ainsi que la valeur moyenne du pH calculé.

**Tableau 6 détaillant les valeurs de pH mesurées dans le mélange contenant la résine 5**

| Temps (minutes) | 0 | 0,5 | 1 | 2 | 3 | 5 | 10 | 20 |
|---|---|---|---|---|---|---|---|---|
| pH E1 | 2,53 | 2,75 | 2,83 | 2,95 | 3,03 | 3,16 | 3,38 | 3,62 |
| pH E2 | 2,53 | 2,79 | 2,86 | 2,95 | 3,03 | 3,16 | 3,37 | 3,58 |
| pH moyen | 2,53 | 2,77 | 2,85 | 2,95 | 3,03 | 3,16 | 3,38 | 3,60 |

Pour la résine 5, une vitesse d'échange initiale de 0,13 unité de pH/minute a été déterminée.

Au vu de ces tableaux 2 à 6 et des vitesses d'échange initiales pour les résines à 1 à 5, on relève que :
- les essais réalisés avec la résine 4 ne permettent pas d'atteindre la valeur de pH cible de 3,5 : à l'issue des essais, le pH stagne à une valeur de 3,22 ;
- seuls les essais réalisés avec les résines 1 et 2 dépassent largement cette valeur cible de pH de 3,5 ;
- les essais réalisé avec les résines 3 et 5 atteignent tout juste cette valeur cible de pH de 3,5 (avec respectivement des valeurs de 3,51 et 3,6) ;
- les résines 1 et 2 ont la meilleure vitesse d'échange initiale et permettent d'atteindre le pH le plus élevé à l'issue de la désacidification dans un bêcher.

Les expérimentations des parties B) et C) qui suivent ont été réalisée uniquement avec les résines 1 et 2.

Les expérimentations B) et C) mettent en œuvre la désacidification du jus de canneberge avec une colonne remplie d'une résine échangeuse d'anions (résines 1 ou 2).

Le débit de circulation du jus de canneberge dans la colonne est ci-après exprimé en mL/minute, mais également en BV/heure.

Pour toutes les expérimentations des parties B et C, le volume de la résine était de 50 mL, à l'exception des 4^{ième} et 5^{ième} expérimentations avec la résine 1 de la partie C) pour lesquelles le volume de résine était de 460 mL.

### B - EXEMPLES COMPARATIFS : DESACIDIFICATION DU JUS DE CANNEBERGE PAR PASSAGE UNIQUE DANS UNE COLONNE D'ECHANGES D'ANIONS (RESINES 1 ET 2) AVEC UN DEBIT DE CIRCULATION DE 5BV/ HEURE:

Ces expérimentations réalisées avec une colonne d'échanges d'anions sans recirculation ont permis de déterminer la capacité d'échange des résines 1 et 2 sur le jus de canneberge tel que décrit ci-dessus.

Le protocole expérimental a été le suivant :
Les résines 1 et 2 ont été chargées chacune dans une colonne. Le jus de canneberge a été percolé au travers le lit de la résine de manière à être désacidifié.

La figure 1 représente de manière schématique le dispositif 1 dans lequel ont été effectuées ces expérimentations de la partie B.

Le débit de circulation du jus de canneberge dans la colonne était toujours de 5 BV/heure (soit 4 mL/minute) pour ces expérimentations réalisées avec la résine 1 et la résine 2.

Le débit de circulation étant constant à 5 BV/heure, lesdites expérimentations de désacidification du jus de canneberge de cette partie B sont des expérimentations comparatives par rapport au procédé de désacidification selon l'invention.

Le tableau 7 détaille ci-dessous pour l'expérimentation mise en œuvre avec la résine 1, en fonction du volume (exprimé en L) de jus de canneberge passé au travers la colonne :
- le BV ;
- l'indice de concentration exprimé en degrés Brix ;
- pHB : les valeurs de pH du jus de canneberge désacidifié mesurées en sortie de colonne 8a par le pH-mètre 18 ;
- pHA : les valeurs de pH du jus de canneberge désacidifié qui ont été mesurées dans le récipient de réception dudit jus (non représenté sur la figure 1).

**Tableau 7 détaillant les valeurs de BV, indice de concentration en degrés Brix, pHA et pHB pour l'expérimentation réalisée avec la résine 1**

| Volume (L) | BV | °Bx | pHB | pHA |
|---|---|---|---|---|
| Jus de canneberge à désacidifier | | 7,6 | | 2,47 |
| 50 | 1 | 1,4 | 9,41 | 9,41 |
| 100 | 2 | 4,3 | 9,31 | 9,32 |
| 150 | 3 | 4,6 | 9,21 | 9,21 |
| 200 | 4 | 4,8 | 9,11 | 9,11 |
| 250 | 5 | 4,9 | 9,01 | 9,02 |
| 300 | 6 | 5,2 | 8,67 | 8,87 |
| 350 | 7 | 5,3 | 4,53 | 7,72 |
| 400 | 8 | 5,7 | 3,62 | 4,58 |
| 450 | 9 | 6,1 | 3,29 | 3,99 |
| 500 | 10 | 6,2 | 3,14 | 3,74 |
| 550 | 11 | 6,7 | 3,00 | 3,55 |
| 600 | 12 | 6,9 | 2,91 | 3,41 |
| 650 | 13 | 7 | 2,84 | 3,32 |
| 700 | 14 | 7,1 | 2,80 | 3,21 |
| 750 | 15 | 7,1 | 2,76 | 3,16 |
| 800 | 16 | 7,2 | 2,73 | 3,11 |
| 850 | 17 | 7,3 | 2,70 | 3,08 |
| 900 | 18 | 7,3 | 2,67 | 3,01 |

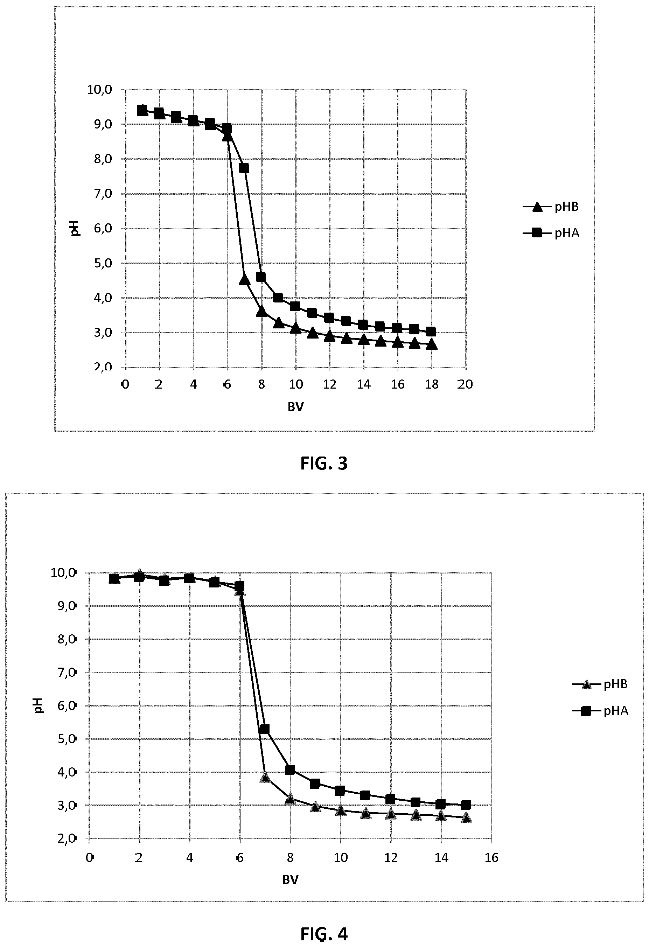

La figure 3 est un graphique de l'évolution des valeurs de pHA et pHB en fonction du BV.

Au vu du tableau 7 et de la figure 3, on relève une diminution constante des valeurs de pHA et pHB.

Les valeurs de pHA sont toujours supérieures ou égales à celles de pHB. Cela s'explique logiquement car :
- pHA représente un pH « moyen » du jus de canneberge, et ce du fait qu'il est mesuré dans le récipient recueillant tout au long de l'expérimentation le jus de canneberge après son passage unique par la colonne, et
- pHB représente un pH « instantané » du jus de canneberge qui est mesuré juste en sortie de colonne.

Les valeurs de pHA et pHB sont supérieures à 9 pendant 5 BV, soit pendant environ la 1^{ière} heure de l'expérimentation.

Au cours de l'expérimentation, la couleur du jus de canneberge en sortie de colonne était noire, puis est devenue verte, bleue et enfin rouge. La quantité de couleur fixée apparaît élevée.

Le jus de canneberge dans le récipient de réception du jus de canneberge désacidifié a atteint la valeur cible de 3,5 après 11 BV (pHA à 11 BV : 3,55 et pHA à 12 BV : 3,41).

Pour ce jus de canneberge recueilli dans le récipient de réception du jus de canneberge désacidifié, la chute de pH est observée entre 7 et 8 BV (pHA à 6 BV : 8,87 et pHA à 7 BV : 7,72).

Au cours de cette expérimentation, le jus de canneberge a donc été soumis à une variation importante du pH ; ce qui a entraîné la modification de la couleur et la précipitation (autrement dit l'altération) de composés d'intérêt tels que les anthocyanes.

Le tableau 8 ci-dessous détaille :
- les concentrations des acides malique et quinique présents dans le jus de canneberge :
- brut (c'est-à-dire le jus de canneberge à désacidifier) ;
- désacidifié dans le récipient de réception lorsque 12 BV de jus de canneberge brut étaient passés au travers la colonne 2 remplie de la résine 1;
- désacidifié dans le récipient de réception lorsque 18 BV de jus de canneberge brut étaient passés au travers la colonne 2 remplie de la résine 1;

- le ratio de la concentration d'acide quinique sur celle de l'acide malique.

**Tableau 8 détaillant les concentrations des acides malique et quinique présents dans le jus de canneberge brut, à 12 BV et 18 BV**

| | g/L | | |
|---|---|---|---|
| Jus de canneberge | acide malique | acide quinique | ratio acide quinique/acide malique |
| brut | 9,47 | 6,63 | 0,70 |
| Désacidifié (12 BV) | 4,89 | 4,46 | 0,91 |
| Désacidifié (18 BV) | 6,74 | 6,07 | 0,90 |

Au vu du tableau 8, on relève qu'au cours de cette expérimentation de désacidification avec la résine 1 :
- les acides quinique et malique sont capturés par la résine 1 : leur concentration passe respectivement de 6,63 à 4,46 et de 9,47 à 4,89 lorsque le jus de canneberge est désacidifié jusqu'à 12 BV, c'est-à-dire juste après avoir atteint la valeur cible de 3,5 ;
- l'acide malique est plus fixé par la résine 1 que l'acide quinique. Le ratio passe de 0,7 à 0,9. L'acide quinique a donc moins d'affinité avec la résine 1.

Le tableau 9 détaille ci-dessous pour l'expérimentation mise en œuvre avec la résine 2, en fonction du volume (exprimé en L) de jus de canneberge passé au travers la colonne :
- le BV ;
- l'indice de concentration exprimé en degrés Brix ;
- pHB tel que défini ci-dessus ;
- pHA tel que défini ci-dessus.

**Tableau 9 détaillant les valeurs de BV, indice de concentration en degrés Brix, pHA et pHB pour l'expérimentation réalisée avec la résine 2**

| Volume(L) | BV | °Bx | pHB | pHA |
|---|---|---|---|---|
| Jus de canneberge à désacidifier | | 7,6 | | 2,47 |
| 50 | 1 | 1,2 | 9,84 | 9,84 |
| 100 | 2 | 4,9 | 9,95 | 9,88 |
| 150 | 3 | 5,1 | 9,82 | 9,78 |
| 200 | 4 | 5,1 | 9,86 | 9,85 |
| 250 | 5 | 5,1 | 9,74 | 9,73 |
| 300 | 6 | 5,1 | 9,47 | 9,62 |
| 350 | 7 | 5,4 | 3,85 | 5,31 |
| 400 | 8 | 6,3 | 3,20 | 4,08 |
| 450 | 9 | 6,9 | 2,97 | 3,67 |
| 500 | 10 | 7,3 | 2,85 | 3,46 |
| 550 | 11 | 7,3 | 2,77 | 3,32 |
| 600 | 12 | 7,2 | 2,75 | 3,21 |
| 650 | 13 | 7,2 | 2,72 | 3,11 |
| 700 | 14 | 7,2 | 2,69 | 3,05 |
| 750 | 15 | 7,3 | 2,64 | 3,02 |

La figure 4 est un graphique de l'évolution des valeurs de pHA et pHB en fonction du BV.

Au vu du tableau 9 et de la figure 4, on relève une diminution constante des valeurs de pHA et pHB.

Les valeurs de pHA et pHB sont supérieures à 9 pendant 6 BV, soit pendant plus de la 1^{ière} heure de l'expérimentation.

Au cours de l'expérimentation, la couleur du jus de canneberge en sortie de colonne était noire, puis est devenue verte, bleue et enfin rouge. La quantité de couleur fixée apparaît élevée.

Le jus de canneberge dans le récipient de réception a atteint la valeur cible de 3,5 après 10 BV (pHA à 9 BV : 3,67 et pHA à 10 BV : 3,46).

Pour ce jus de canneberge recueilli dans le récipient de réception, la chute de pH est observée entre 6 et 7 BV (pHA à 6 BV : 9,62 et pHA à 7 BV : 5,31).

Au cours de cette expérimentation, le jus de canneberge a donc été soumis à une variation importante du pH ; ce qui a entraîné la modification de la couleur et la précipitation (autrement dit l'altération) de composés d'intérêt tels que les anthocyanes.

Le tableau 10 ci-dessous détaille :
- les concentrations des acides malique et quinique présents dans le jus de canneberge :
- brut (c'est-à-dire le jus de canneberge à désacidifier) ;
- désacidifié dans le récipient de réception lorsque 10 BV de jus de canneberge brut étaient passés au travers la colonne remplie de la résine 2 ;
- désacidifié dans le récipient de réception lorsque 15 BV de jus de canneberge brut étaient passés au travers la colonne remplie de la résine 2.

- le ratio de la concentration d'acide quinique sur celle de l'acide malique.

**Tableau 10 détaillant les concentrations des acides malique et quinique dans le jus de canneberge brut, à 10 BV et 15 BV**

| | g/L | | |
|---|---|---|---|
| Jus de canneberge | acide malique | acide quinique | ratio acide quinique/acide malique |
| brut | 9,47 | 6,63 | 0,70 |
| Désacidifié (10 BV) | 5,11 | 5,91 | 1,16 |
| Désacidifié (15 BV) | 8,29 | 6,90 | 0,83 |

Au vu du tableau 10, on relève qu'au cours de cette expérimentation de désacidification avec la résine 2 :
- les acides quinique et malique sont capturés par la résine 2 : leur concentration passe respectivement de 6,63 à 5,91 et de 9,47 à 5,11 lorsque le jus de canneberge est désacidifié jusqu'à 10 BV, c'est-à-dire juste après avoir atteint la valeur cible de 3,5 ;
- l'acide malique est plus fixé par la résine 2 que l'acide quinique. Le ratio passe de 0,7 à 0,83. L'acide quinique a donc moins d'affinité avec la résine 2.

En comparant les tableaux 8 et 10, on relève que :
- la résine 2 capture beaucoup moins l'acide quinique que la résine 1 ;
- la résine 2 a un peu moins de capacité d'échange que la résine 1. En effet, elle capture moins les acides malique et quinique que la résine 1.

### C- DESACIDIFICATION DU JUS DE CANNEBERGE PAR CIRCULATION EN BOUCLE DANS UNE COLONNE D'ECHANGES D'ANIONS:

Les expérimentations de cette partie C ont été réalisées dans une colonne d'échanges d'anions avec circulation en boucle. Les résines 1 et 2 ont été chacune chargées dans une colonne.

En sortie de colonne, l'effluent de jus de canneberge était réintroduit dans le bac d'alimentation.

La figure 2a représente de manière schématique le dispositif 10 dans lequel ont été effectuées ces expérimentations avec circulation en boucle du jus de canneberge à désacidifier.

Avec une colonne remplie de la résine 1, cinq expérimentations ont été réalisées de manière à varier la charge de jus de canneberge à désacidifier (c'est-à-dire le volume de jus de canneberge à désacidifier mis en circulation en boucle).

Le pH mesuré du jus de canneberge désacidifié mesuré en sortie de colonne 8b par le pH-mètre 18 est ci-après dénommé « pH2 ».

Le pH mesuré du jus de canneberge mesuré dans la cuve 2 par un pH-mètre non représenté sur la figure 2a est ci-après dénommé « pHl ».

La 1^{ière} expérimentation a été réalisée avec une charge de jus de canneberge de 8 BV.

Pour cette 1^{ière} expérimentation, le tableau 11 ci-dessous détaille en fonction du temps (exprimé en minutes) :
- les valeurs de pH1 et pH2 ;
- le débit de jus de canneberge circulant dans la colonne (exprimé en mL/minutes et en BV/heure). Le débit est toujours compris entre 10 BV/heure et 250 BV/heure.

**Tableau 11 détaillant les valeurs de pH2, pH1 et du débit de circulation jus de canneberge avec une charge de 8 BV**

| Temps (minutes) | pH2 | pH1 | Débit (mL/minute) | Débit (BV/heure) |
|---|---|---|---|---|
| 1 | 5,01 | 2,56 | 90 | 108,0 |
| 2 | 4,09 | 2,57 | 90 | 108,0 |
| 4 | 3,2 | 2,72 | 63 | 75,6 |
| 5 | 3,29 | 2,78 | 45 | 54,0 |
| 7 | 3,61 | 2,8 | 27 | 32,4 |
| 8 | 3,6 | 2,86 | 27 | 32,4 |
| 9 | 3,58 | 2,88 | 18 | 21,6 |
| 10 | 3,58 | 2,88 | 18 | 21,6 |
| 11 | 3,65 | 2,89 | 18 | 21,6 |
| 12 | 3,77 | 2,91 | 18 | 21,6 |
| 13 | 3,82 | 2,93 | 18 | 21,6 |
| 15 | 3,84 | 2,96 | 18 | 21,6 |
| 17 | 3,86 | 3,00 | 18 | 21,6 |
| 19 | 3,84 | 3,04 | 22,5 | 27,0 |
| 21 | 3,72 | 3,08 | 27 | 32,4 |
| 23 | 3,69 | 3,16 | 27 | 32,4 |
| 25 | 3,69 | 3,16 | 27 | 32,4 |
| 27 | 3,7 | 3,2 | 27 | 32,4 |
| 30 | 3,72 | 3,25 | 27 | 32,4 |
| 35 | 3,75 | 3,32 | 27 | 32,4 |
| 36 | 3,75 | 3,36 | 36 | 43,2 |
| 40 | 3,74 | 3,39 | 45 | 54,0 |
| 44 | 3,77 | 3,46 | 45 | 54,0 |
| 47 | 3,79 | 3,5 | 54 | 64,8 |

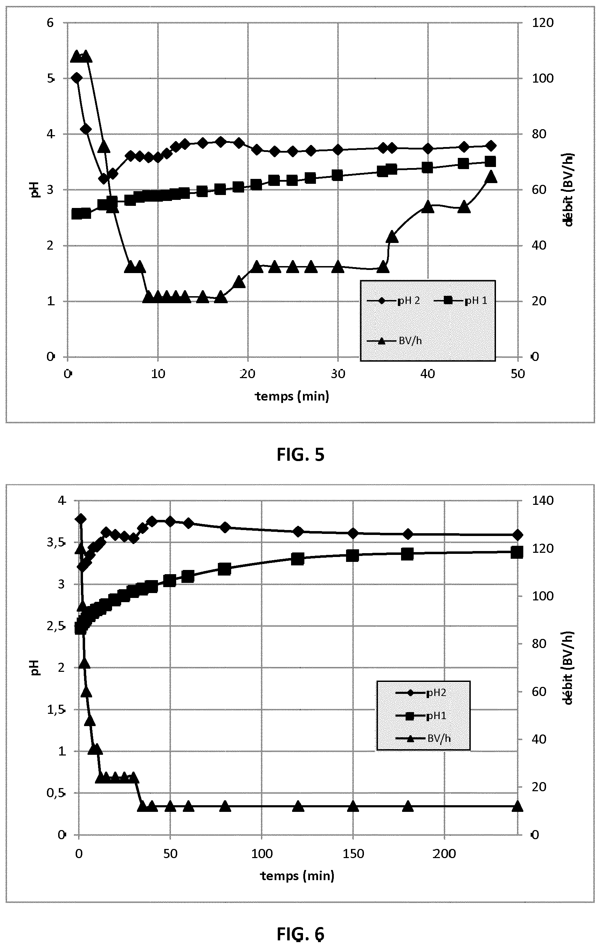

La figure 5 est un graphique représentant l'évolution de pH1, pH2 et du débit exprimé en BV/heure en fonction du temps pour cette 1^{ière} expérimentation avec circulation en boucle avec une charge de jus de canneberge de 8 BV.

Au vu du tableau 11 et de la figure 5, on relève notamment que :
- le pH du jus de canneberge dans le récipient a atteint la valeur cible de 3,5 au bout de 47 minutes de circulation en boucle ; Cette 1^{ière} expérimentation correspond ainsi à une mise en œuvre du procédé de désacidification selon l'invention ;
- aux temps 1 minute, puis 2 minutes, le pH du jus de canneberge en sortie de colonne était respectivement de 5,01, puis 4,09 ; ensuite après 4 minutes, il était de 3,2.
- le jus de canneberge a toujours eu en sortie de colonne un pH inférieur ou sensiblement égal à 5. Le procédé selon l'invention n'est donc pas susceptible d'altérer les composés d'intérêt tels que les anthocyanes.

La 2^{ième} expérimentation a été réalisée avec une charge de jus de canneberge de 10 BV.

Pour cette 2^{ième} expérimentation, le tableau 12 ci-dessous détaille en fonction du temps (exprimé en minutes) :
- les valeurs de pH1 et pH2 ;
- le débit de jus de canneberge circulant dans la colonne (exprimé en mL/minutes et en BV/heure). Le débit est toujours compris entre 10 BV/heure et 250 BV/heure.

**Tableau 12 détaillant les valeurs de pH2, pH1 et du débit de circulation jus de canneberge avec une charge de 10 BV**

| Temps (minutes) | pH2 | pH1 | Débit (mL/minute) | Débit (BV/heure) |
|---|---|---|---|---|
| 1 | 3,78 | 2,48 | 100 | 120 |
| 2 | 3,21 | 2,53 | 80 | 96 |
| 3 | 3,24 | 2,56 | 60 | 72 |
| 4 | 3,26 | 2,59 | 50 | 60 |
| 6 | 3,35 | 2,63 | 40 | 48 |
| 8 | 3,44 | 2,67 | 30 | 36 |
| 10 | 3,45 | 2,70 | 30 | 36 |
| 12 | 3,5 | 2,72 | 20 | 24 |
| 15 | 3,62 | 2,76 | 20 | 24 |
| 20 | 3,59 | 2,82 | 20 | 24 |
| 25 | 3,57 | 2,87 | 20 | 24 |
| 30 | 3,55 | 2,92 | 20 | 24 |
| 35 | 3,67 | 2,95 | 10 | 12 |
| 40 | 3,75 | 2,98 | 10 | 12 |
| 50 | 3,75 | 3,05 | 10 | 12 |
| 60 | 3,73 | 3,10 | 10 | 12 |
| 80 | 3,68 | 3,19 | 10 | 12 |
| 120 | 3,63 | 3,31 | 10 | 12 |
| 150 | 3,61 | 3,35 | 10 | 12 |
| 180 | 3,6 | 3,37 | 10 | 12 |
| 240 | 3,59 | 3,39 | 10 | 12 |

La figure 6 est un graphique représentant l'évolution de pH1, pH2 et du débit exprimé en BV/heure en fonction du temps pour cette 2^{ième} expérimentation avec circulation en boucle avec une charge de jus de canneberge de 10 BV.

Au vu du tableau 12 et de la figure 6, on relève notamment que :
- le jus de canneberge a toujours eu en sortie de colonne un pH inférieur à 4. Le procédé selon l'invention n'est donc pas susceptible d'altérer les composés d'intérêt tels que les anthocyanes.
- au bout de 240 minutes d'expérimentation, le pH du jus de canneberge dans le récipient a atteint une valeur sensiblement constante de l'ordre de 3,39, qui est donc très proche de la valeur cible de 3,5 fixée ;
- cette 2^{ième} expérimentation correspond ainsi à une mise en œuvre du procédé de désacidification selon l'invention.

La 3^{ième} expérimentation a été réalisée avec une charge de jus de canneberge de 9 BV.

Pour cette 3^{ième} expérimentation, le tableau 13 ci-dessous détaille en fonction du temps (exprimé en minutes) :
- les valeurs de pH1 et pH2 ;
- le débit de jus de canneberge circulant dans la colonne (exprimé en mL/minutes et en BV/heure). Le débit est toujours compris entre 10 BV/heure et 250 BV/heure.

**Tableau 13 détaillant les valeurs de pH2, pH1 et du débit de circulation jus de canneberge avec une charge de 9BV**

| Temps (minutes) | pH2 | pH1 | Débit (mL/minute) | Débit (BV/heure) |
|---|---|---|---|---|
| 1 | 4,09 | 2,50 | 100 | 120 |
| 1,5 | 3,33 | 2,52 | 70 | 84 |
| 3 | 3,37 | 2,58 | 50 | 60 |
| 5 | 3,41 | 2,64 | 40 | 48 |
| 7 | 3,48 | 2,69 | 30 | 36 |
| 10 | 3,49 | 2,76 | 30 | 36 |
| 15 | 3,68 | 2,83 | 30 | 36 |
| 20 | 3,65 | 2,91 | 20 | 24 |
| 30 | 3,63 | 3,04 | 20 | 24 |
| 50 | 3,66 | 3,24 | 20 | 24 |
| 70 | 3,68 | 3,44 | 20 | 24 |
| 90 | 3,71 | 3,44 | 20 | 24 |
| 110 | 3,71 | 3,50 | 30 | 36 |

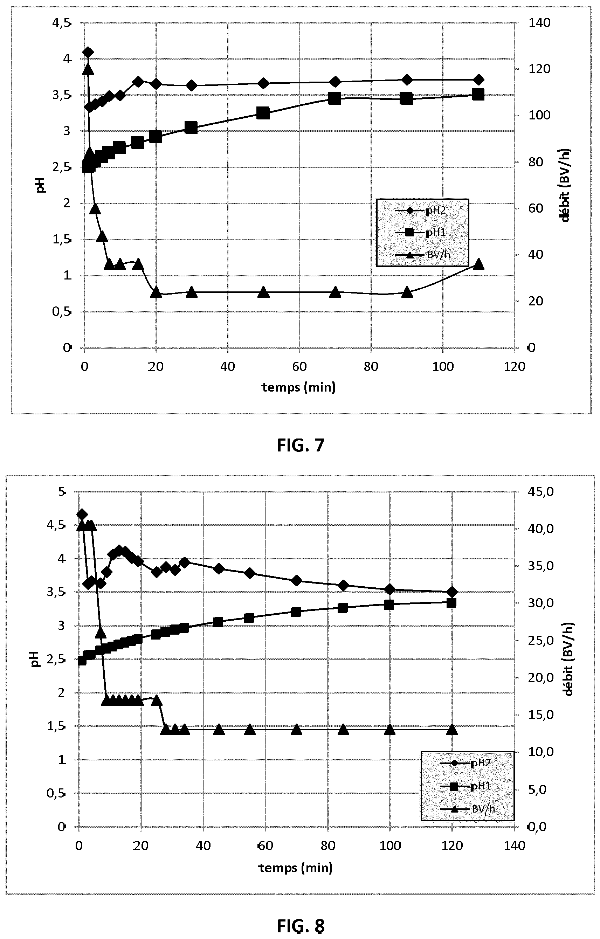

La figure 7 est un graphique représentant l'évolution de pH1, pH2 et du débit exprimé en BV/heure en fonction du temps pour cette 3^{ième} expérimentation avec circulation en boucle avec une charge de jus de canneberge de 9 BV.

Au vu du tableau 13 et de la figure 7, on relève notamment que :
- le jus de canneberge a toujours eu en sortie de colonne un pH inférieur ou sensiblement égal à 4. Le procédé selon l'invention n'a donc pas altéré les composés d'intérêt tels que les anthocyanes.
- au bout de 110 minutes d'expérimentation, le pH du jus de canneberge dans le récipient a atteint la valeur cible de 3,5 ;
- cette 3^{ième} expérimentation correspond ainsi à une mise en œuvre du procédé de désacidification selon l'invention.

La 4^{ième} expérimentation a été réalisée avec une charge de jus de canneberge de 9 BV.

Pour cette 4^{ième} expérimentation, le tableau 14 ci-dessous détaille en fonction du temps (exprimé en minutes) :
- les valeurs de pH1 et pH2 ;
- le débit de jus de canneberge circulant dans la colonne (exprimé en mL/minutes et en BV/heure). Le débit est toujours compris entre 10 BV/heure et 250 BV/heure.

**Tableau 14 détaillant les valeurs de pH2, pH1 et du débit de circulation jus de canneberge avec une charge de 9BV**

| Temps (minutes) | pH2 | pH1 | Débit (mL/minute) | Débit (BV/heure) |
|---|---|---|---|---|
| 1 | 4,66 | 2,49 | 310 | 40,4 |
| 3 | 3,62 | 2,57 | 310 | 40,4 |
| 4 | 3,66 | 2,58 | 310 | 40,4 |
| 7 | 3,63 | 2,64 | 200 | 26,1 |
| 9 | 3,8 | 2,67 | 130 | 17,0 |
| 11 | 4,06 | 2,7 | 130 | 17,0 |
| 13 | 4,12 | 2,73 | 130 | 17,0 |
| 15 | 4,1 | 2,76 | 130 | 17,0 |
| 17 | 4,01 | 2,78 | 130 | 17,0 |
| 19 | 3,96 | 2,81 | 130 | 17,0 |
| 25 | 3,8 | 2,88 | 130 | 17,0 |
| 28 | 3,87 | 2,92 | 100 | 13,0 |
| 31 | 3,83 | 2,95 | 100 | 13,0 |
| 34 | 3,94 | 2,97 | 100 | 13,0 |
| 45 | 3,85 | 3,06 | 100 | 13,0 |
| 55 | 3,78 | 3,12 | 100 | 13,0 |
| 70 | 3,67 | 3,21 | 100 | 13,0 |
| 85 | 3,6 | 3,27 | 100 | 13,0 |
| 100 | 3,54 | 3,32 | 100 | 13,0 |
| 120 | 3,5 | 3,35 | 100 | 13,0 |

La figure 8 est un graphique représentant l'évolution de pH1, pH2 et du débit exprimé en BV/heure en fonction du temps pour cette 4^{ième} expérimentation avec circulation en boucle avec une charge de jus de canneberge de 9 BV.

Au vu du tableau 14 et de la figure 8, on relève notamment que :
- le jus de canneberge a toujours eu en sortie de colonne un pH inférieur à 5. Le procédé selon l'invention n'est donc pas susceptible d'altérer les composés d'intérêt tels que les anthocyanes.
- au bout de 120 minutes d'expérimentation, le pH du jus de canneberge dans le récipient a atteint une valeur sensiblement constante de l'ordre de 3,35, qui est donc très proche de la valeur cible de 3,5 fixée ;
- cette 4^{ième} expérimentation correspond ainsi à une mise en œuvre du procédé de désacidification selon l'invention.

La 5^{ième} expérimentation a été réalisée avec une charge de 8 BV. Pour cette 5^{ième} expérimentation, le tableau 15 ci-dessous détaille en fonction du temps (exprimé en minutes) : les valeurs de pH1 et pH2 et le débit de jus de canneberge circulant dans la colonne (exprimé en mL/minutes et en BV/heure).

Dans cette 5^{ième} expérimentation, pendant les deux premières minutes, le débit de circulation du jus de canneberge dans la colonne était de 2,6 BV/heure, donc inférieur à 10 BV/heure.

Ainsi, pendant les deux premières minutes de cette 5^{ième} expérimentation, le procédé de désacidification selon l'invention n'a pas été mis en œuvre. Nous détaillons ci-dessous les conséquences que cela a eues sur le jus de canneberge à désacidifier.

**Tableau 15 détaillant les valeurs de pH2, pH1 et du débit de circulation jus de canneberge avec une charge de 8 BV**

| Temps (minutes) | pH2 | pH1 | Débit (mL/minute) | Débit (BV/heure) |
|---|---|---|---|---|
| 1 | 8,66 | 2,52 | 20 | 2,6 |
| 2 | 8,92 | 2,54 | 20 | 2,6 |
| 3 | 3,28 | 2,65 | 400 | 52,2 |
| 4 | 3,33 | 2,68 | 300 | 39,1 |
| 6 | 3,39 | 2,72 | 200 | 26,1 |
| 10 | 3,56 | 2,81 | 200 | 26,1 |
| 14 | 3,55 | 2,90 | 200 | 26,1 |
| 16 | 3,54 | 2,99 | 200 | 26,1 |
| 18 | 3,55 | 3,03 | 200 | 26,1 |
| 20 | 3,55 | 2,90 | 200 | 26,1 |
| 24 | 3,56 | 3,10 | 200 | 26,1 |
| 28 | 3,53 | 3,18 | 230 | 30,0 |
| 30 | 3,54 | 3,22 | 230 | 30,0 |
| 34 | 3,54 | 3,28 | 260 | 33,9 |
| 37 | 3,56 | 3,33 | 260 | 33,9 |
| 40 | 3,57 | 3,38 | 300 | 39,1 |
| 44 | 3,58 | 3,44 | 350 | 45,7 |
| 45 | 3,59 | 3,45 | 350 | 45,7 |
| 49 | 3,6 | 3,50 | 400 | 52,2 |

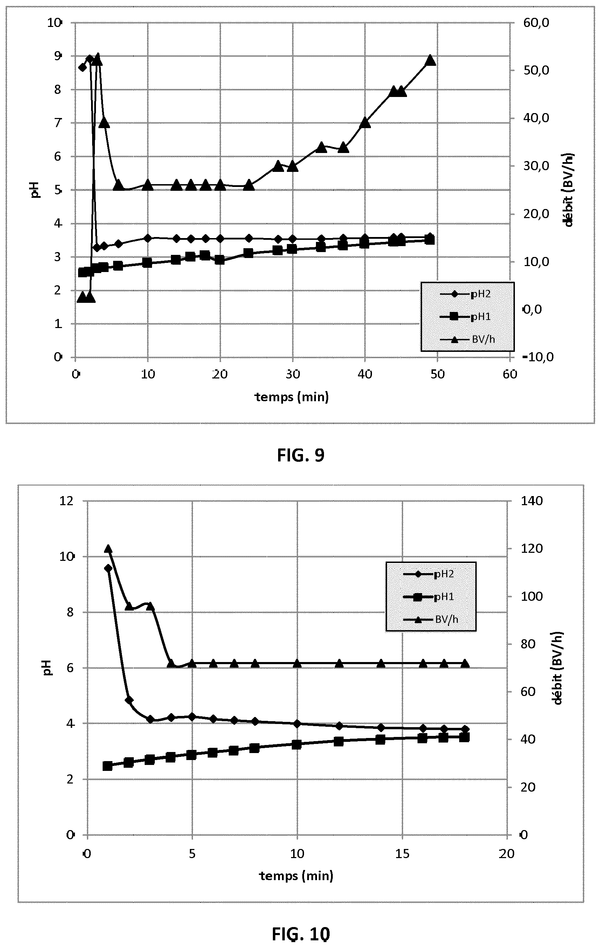

La figure 9 est un graphique représentant l'évolution de pH1, pH2 et du débit exprimé en BV/heure en fonction du temps pour cette 5^{ième} expérimentation avec circulation en boucle avec une charge de jus de canneberge de 9 BV.

Au vu du tableau 15 et de la figure 9, on relève notamment que :
- le fait que pendant les deux premières minutes de l'expérimentation (c'est-à-dire de la circulation en boucle du jus de canneberge), le débit était seulement de 2,6 BV/heure, cela a eu un impact sur la valeur du pH du jus de canneberge en sortie de colonne en début d'expérimentation qui était de plus de 8,5 (à savoir une valeur de pH à laquelle les composés d'intérêt tels que les anthocyanes sont très susceptibles d'être altérés) ;
- dès que le débit a été augmenté passant de 2,6 BV/heure à 52,2 BV/heure, le pH du jus de canneberge est immédiatement passé de 8,92 à 3,28, c'est-à-dire le jus de canneberge a atteint une valeur de pH à laquelle les composés d'intérêt ne sont pas altérés.
- au bout de 49 minutes d'expérimentation, le pH du jus de canneberge dans le récipient a atteint la valeur cible de 3,5.

Ainsi, cette 5^{ième} expérimentation témoigne de l'importance que le débit de circulation du jus de canneberge doit être d'au moins 10 BV/heure, mais également de sa régulation, et tout particulièrement en début d'expérimentation où il doit être élevé pour que le jus de canneberge n'ait pas en sortie de colonne un pH supérieur aux valeurs auxquelles les composés d'intérêt tels que les anthocyanes sont susceptibles d'être altérés.

Avec une colonne remplie de la résine 2, trois expérimentations ont été réalisées de manière à varier la charge de jus de canneberge à désacidifier (c'est-à-dire mis en circulation en boucle).

La 1^{ière} expérimentation a été réalisée avec une charge de 6 BV.

Pour cette 1^{ière} expérimentation, le tableau 16 ci-dessous détaille en fonction du temps (exprimé en minutes) :
- les valeurs de pH1 et pH2 ;
- le débit de jus de canneberge circulant dans la colonne (exprimé en mL/minutes et en BV/heure). Le débit est toujours compris entre 10 BV/heure et 250 BV/heure.

**Tableau 16 détaillant les valeurs de pH2, pH1 et du débit de circulation jus de canneberge avec une charge de 6 BV**

| Temps (minutes) | pH2 | pH1 | Débit (mL/minute) | Débit (BV/heure) |
|---|---|---|---|---|
| 1 | 9,58 | 2,50 | 100 | 120 |
| 2 | 4,84 | 2,62 | 80 | 96 |
| 3 | 4,15 | 2,72 | 80 | 96 |
| 4 | 4,21 | 2,81 | 60 | 72 |
| 5 | 4,24 | 2,89 | 60 | 72 |
| 6 | 4,16 | 2,97 | 60 | 72 |
| 7 | 4,11 | 3,04 | 60 | 72 |
| 8 | 4,07 | 3,13 | 60 | 72 |
| 10 | 3,99 | 3,26 | 60 | 72 |
| 12 | 3,91 | 3,37 | 60 | 72 |
| 14 | 3,85 | 3,44 | 60 | 72 |
| 16 | 3,82 | 3,49 | 60 | 72 |
| 17 | 3,8 | 3,51 | 60 | 72 |
| 18 | 3,79 | 3,52 | 60 | 72 |

La figure 10 est un graphique représentant l'évolution de pH1, pH2 et du débit exprimé en BV/heure en fonction du temps pour cette 1^{ière} expérimentation avec circulation en boucle avec une charge de jus de canneberge de 6 BV.

Au vu du tableau 16 et de la figure 10, on relève notamment que :
- malgré un débit de circulation élevé de 120 BV/heure en début d'expérimentation, la valeur de pH du jus de canneberge en sortie de colonne était de 9,58 (à savoir une valeur de pH à laquelle les composés d'intérêt tels que les anthocyanes sont très susceptibles d'être altérés) ;
- ensuite, après 2 minutes d'expérimentation, les valeurs de pH du jus de canneberge ont toujours été inférieures à 5 (c'est-à-dire des valeurs de pH auxquelles les composés d'intérêt ne sont pas susceptibles d'être altérés) ;
- ainsi, la 1^{ière} minute d'expérimentation, malgré un débit de circulation très élevé, a pu être préjudiciable aux composés d'intérêt ;
- avec une charge de 6 BV de jus de canneberge et la résine 2, un débit de circulation plus élevé que 120 BV/heure et/ou un autre dimensionnement de la colonne devraient être mis en œuvre pour éviter qu'en début d'expérimentation des composés d'intérêt ne soient altérés du fait que le pH du jus de canneberge en sortie de colonne est supérieur à 9 ;
- au bout de 17 minutes d'expérimentation, le pH du jus de canneberge dans le récipient a atteint la valeur cible de 3,5.

La 2^{ième} expérimentation a été réalisée avec une charge de 8 BV.

Pour cette 2^{ième} expérimentation, le tableau 17 ci-dessous détaille en fonction du temps (exprimé en minutes) :
- les valeurs de pH1 et pH2 ;
- le débit de jus de canneberge circulant dans la colonne (exprimé en mL/minutes et en BV/heure). Le débit est toujours compris entre 10 BV/heure et 250 BV/heure.

**Tableau 17 détaillant les valeurs de pH2, pH1 et du débit de circulation jus de canneberge avec une charge de 8 BV**

| Temps (minutes) | pH2 | pH1 | Débit (mL/minute) | Débit (BV/heure) |
|---|---|---|---|---|
| 1 | 6,83 | 2,47 | 100 | 120 |
| 1,5 | 4,18 | 2,50 | 100 | 120 |
| 2 | 3,77 | 2,54 | 100 | 120 |
| 2,5 | 3,68 | 2,59 | 80 | 96 |
| 3 | 3,66 | 2,62 | 80 | 96 |
| 4 | 3,59 | 2,70 | 40 | 48 |
| 5 | 3,62 | 2,75 | 40 | 48 |
| 6 | 3,63 | 2,80 | 30 | 36 |
| 7 | 3,6 | 2,85 | 20 | 24 |
| 8 | 3,59 | 2,90 | 10 | 12 |

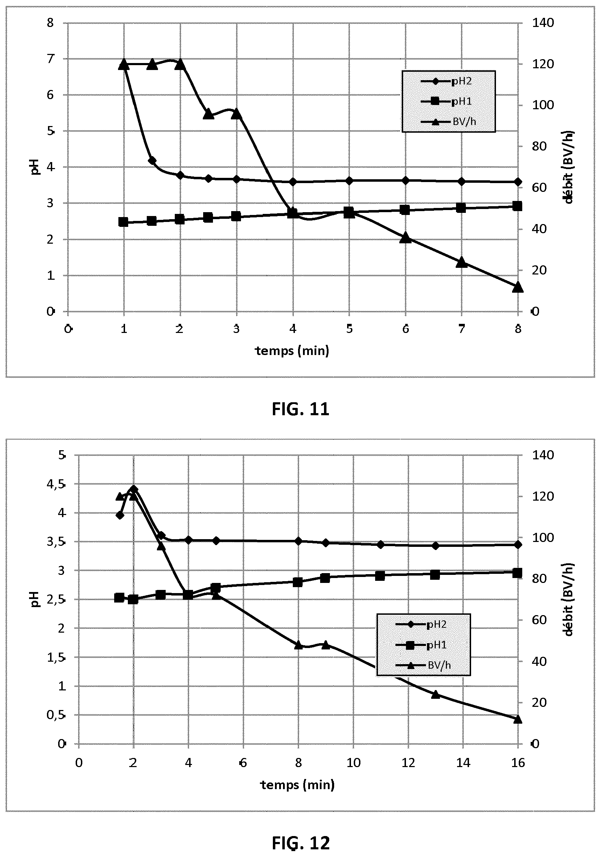

La figure 11 est un graphique représentant l'évolution de pH1, pH2 et du débit exprimé en BV/heure en fonction du temps pour cette 2^{ième} expérimentation avec circulation en boucle avec une charge de jus de canneberge de 8 BV.

Au vu du tableau 17 et de la figure 11, on relève notamment que :
- malgré un débit de circulation élevé de 120 BV/heure en début d'expérimentation, la valeur de pH du jus de canneberge en sortie de colonne était de 6,83 (à savoir une valeur de pH moyennement acceptable car des composés d'intérêt tels que les anthocyanes sont susceptibles d'être altérés) ;
- ensuite, après 1,5 minutes d'expérimentation, les valeurs de pH du jus de canneberge ont toujours été inférieures à 4,55 (c'est-à-dire des valeurs de pH auxquelles les composés d'intérêt ne sont pas susceptibles d'être altérés) ;
- la valeur cible de 3,5 n'a pas été atteinte au cours de cette 2^{ième} expérimentation ;
- ainsi au cours de cette 2^{ième} expérimentation, outre le fait que des composés d'intérêt ont pu être altérés, le débit de circulation du jus de canneberge n'a pas été ajusté de telle sorte que le pH du jus de canneberge augmente jusqu'à la valeur cible de 3,5 qui avait été fixée.

La 3^{ième} expérimentation a été réalisée avec une charge de 10 BV.

Pour cette 3^{ième} expérimentation, le tableau 18 ci-dessous détaille en fonction du temps (exprimé en minutes) :
- les valeurs de pH1 et pH2 ;
- le débit de jus de canneberge circulant dans la colonne (exprimé en mL/minutes et en BV/heure). Le débit est toujours compris entre 10 BV/heure et 250 BV/heure.

**Tableau 18 détaillant les valeurs de pH2, pH1 et du débit de circulation jus de canneberge avec une charge de 10 BV**

| Temps (minutes) | pH2 | pH1 | Débit (mL/minute) | Débit (BV/heure) |
|---|---|---|---|---|
| 1,5 | 3,96 | 2,54 | 100 | 120 |
| 2 | 4,41 | 2,51 | 100 | 120 |
| 3 | 3,61 | 2,59 | 80 | 96 |
| 4 | 3,53 | 2,59 | 60 | 72 |
| 5 | 3,52 | 2,71 | 60 | 72 |
| 8 | 3,51 | 2,81 | 40 | 48 |
| 9 | 3,48 | 2,88 | 40 | 48 |
| 11 | 3,45 | 2,92 | 30 | 36 |
| 13 | 3,43 | 2,94 | 20 | 24 |
| 16 | 3,45 | 2,97 | 10 | 12 |

La figure 12 est un graphique représentant l'évolution de pH1, pH2 et du débit exprimé en BV/heure en fonction du temps pour cette 3^{ième} expérimentation avec circulation en boucle avec une charge de jus de canneberge de 10 BV.

Au vu du tableau 18 et de la figure 12, on relève notamment que :
- le jus de canneberge a toujours eu en sortie de colonne un pH inférieur à 4,5. Le procédé selon l'invention n'est donc pas susceptible d'altérer les composés d'intérêt tels que les anthocyanes ;
- la valeur cible de 3,5 n'a pas été atteinte au cours de cette 3^{ième} expérimentation.
- avec cette 3^{ième} expérimentation, le débit de circulation du jus de canneberge n'a pas été ajusté de telle sorte que le pH du jus de canneberge augmente jusqu'à la valeur cible de 3,5 qui avait été fixée.

Le tableau 19 est un tableau récapitulatif qui détaille selon les expérimentations réalisées (à savoir les expérimentations E1 à E5 avec la résine 1 et les expérimentations E'1 à E'3 avec la résine 2) :
- le volume jus de canneberge mis en circulation en boule pour être désacidifié, autrement dit la charge de jus de canneberge à désacidifier. Ce volume est exprimé en BV ;
- l'indice de concentration exprimé en degrés Brix (°Bx) ;
- le pH final à l'issue de l'expérimentation ;
- la durée de l'expérimentation ;
- la densité optique à 420 nm, 520 nm, 620 nm et 280 nm ;
- l'intensité et la nuance ;
- la densité optique relative à 420 nm, 520 nm, 620 nm et 280 nm ;
- l'intensité relative et la nuance relative ;
- les concentrations en acide malique et en acide quinique dans le jus de canneberge désacidifié ;
- le ratio de ces concentrations en acide malique et en acide quinique.

En outre, dans une colonne intitulée « Jus brut » sont rappelées les caractéristiques du jus de canneberge brut, c'est-à-dire avant sa désacidification.

La densité optique a été mesurée par spectrophotométrie UV- visible

Le paramètre de l'intensité correspond à la somme des valeurs de la densité optique à 420 nm, 520 nm et 620 nm.

Le paramètre de la nuance correspond au ratio de la densité optique à 420 nm sur celle à 520 nm.

La densité optique à 280 nm fournit une indication sur la concentration des cycles carbonés et doubles liaison qui est donc proportionnelle à la concentration des molécules fonctionnelles.

Les densités optiques relatives correspondent aux densités optiques divisées par l'indice de concentration exprimé en degrés Brix du jus de canneberge correspondant. Ces valeurs de densité optique relative tiennent compte de l'effet de dilution du fait de la présence d'eau dans la colonne. Il en est de même pour les valeurs d'intensité relative et de nuance relative.

Les concentrations en acides malique et quinique ont été déterminées par HPLC avec l'appareillage qui a été décrit ci-dessus.

Au cours de toutes ces expérimentations mettant en œuvre le procédé selon l'invention avec les résines 1 et 2, il convient de noter qu'avec une régulation du débit de circulation, le jus de canneberge a pu être désacidifié en passant d'une valeur de pH de 2,47 à des valeurs proches de la valeur cible de 3,5. Le débit de circulation initial doit être très élevé pour que le pH du jus de canneberge en sortie de colonne ne soit pas en début d'expérimentation trop élevé (et donc préjudiciable pour les composés d'intérêt). Ensuite, le débit de circulation est graduellement réduit, puis stabilisé aux environs de 20 BV/heure pour la résine 1 et 70 BV/heure pour la résine 2. La résine 2 a une cinétique plus rapide que la résine 1 ; ce qui requiert de bien réguler le pH du jus de canneberge en sortie de colonne, et tout particulièrement en début d'expérimentation.

**Tableau 19 récapitulatif des résultats des 8 expérimentations mettant en œuvre le procédé de désacidification selon l'invention**

| | | **Résine 1** | | | | | **Résine 2** | | |
|---|---|---|---|---|---|---|---|---|---|
| | **Jus brut** | **E1** | **E2** | **E3** | **E4** | **E5** | **E'1** | **E'2** | **E'3** |
| **Volume de la résine (mL)** | | 50 | 50 | 50 | 460 | 460 | 50 | 50 | 50 |
| **V produit** | | 8 | 10 | 9 | 9 | 8 | 6 | 8 | 10 |
| **Brix** (°B) | 7,6 | 5,4 | 5,4 | 5,3 | 5,4 | 7,4 | 5,0 | 5,6 | 5,9 |
| **pH** | 2,47 | 3,50 | 3,39 | 3,50 | 3,35 | 3,50 | 3,52 | 2,90 | 2,97 |
| **Temps (minutes)** | | 47 | 240 | 110 | 120 | 49 | 18 | 8 | 16 |
| **ac. malique (g/L)** | 9,47 | 5,35 | 6,35 | 5,26 | 4,88 | 6,32 | 4,59 | 5,40 | 6,35 |
| **ac. quinique (g/L)** | 6,63 | 5,05 | 5,55 | 4,92 | 4,85 | 6,01 | 4,00 | 4,73 | 5,55 |
| **ratio acide quinique/acide malique** | 0,70 | 0,94 | 0,87 | 0,94 | 0,99 | 0,95 | 0,87 | 0,88 | 0,87 |
| **Densité optique :** | | | | | | | | | |
| **420 nm** | 1,625 | 1,240 | 1,240 | 1,030 | 1,390 | 1,830 | 0,560 | 0,925 | 1,130 |
| **520 nm** | 2,705 | 2,16 | 2,390 | 1,910 | 2,545 | 2,770 | 1,115 | 2,085 | 2,675 |
| **620 nm** | 0,230 | 0,175 | 0,160 | 0,150 | 0,220 | 0,279 | 0,065 | 0,065 | 0,090 |
| **280 nm** | 29,320 | - | - | - | - | 25,020 | - | - | - |
| **intensité** | 4,560 | 3,580 | 3,790 | 3,090 | 4,160 | 4,880 | 1,740 | 3,080 | 3,900 |
| **nuance** | 0,600 | 0,570 | 0,520 | 0,540 | 0,550 | 0,660 | 0,500 | 0,440 | 0,420 |
| **Densité optique relative :** | | | | | | | | | |
| **420 nm** | 0,21 | 0,23 | 0,23 | 0,19 | 0,26 | 0,25 | 0,11 | 0,17 | 0,19 |
| **520 nm** | 0,36 | 0,40 | 0,44 | 0,36 | 0,47 | 0,37 | 0,22 | 0,37 | 0,45 |
| **620 nm** | 0,03 | 0,03 | 0,03 | 0,03 | 0,04 | 0,04 | 0,01 | 0,01 | 0,02 |
| **280 nm** | 3,86 | - | - | - | - | 3,38 | - | - | - |
| **intensité relative** | 0,60 | 0,66 | 0,70 | 0,58 | 0,77 | 0,66 | 0,29 | 0,14 | 0,11 |
| **nuance relative** | 0,08 | 0,11 | 0,10 | 0,10 | 0,10 | 0,09 | 0,10 | 0,08 | 0,07 |

Au vu du tableau 19, nous relevons que :
- la résine 1 a une capacité d'échange 50% plus importante que la résine 2. En effet, avec la résine 1, il est possible de désacidifier environ jusqu'à 9 BV du jus de canneberge en maintenant le pH à 3,5, alors qu'il faut limiter la charge à 6 BV avec la résine 2 pour garantir le niveau de 3,5 unités pH ;
- dans ces conditions, la résine 2 entraîne une diminution de l'intensité colorée importante : le jus brut étant à 0,60 et atteint 0,77 à pH 3,5 avec la résine 1 mais seulement 0,29 avec la résine 2 ;
- le procédé selon l'invention augmente le ratio de la concentration de l'acide quinique sur celle de l'acide malique : il passe de 0,7 à 0,9- 1 dans le jus désacidifié.
- l'acide malique diminue en concentration nettement plus que l'acide quinique.

Selon le procédé de l'invention, les jus obtenus de 10 essais ont été mélangés et concentrés à 50 Brix. Le jus désacidifié concentré est comparé au jus concentré brut.
Nous constatons :
- une diminution de 20% de l'acide malique
- une concentration constante en acide quinique
- la quantité des cations (comme le sodium, le potassium, le magnésium et le calcium) n'a pas varié au cours du procédé.

Les proanthocyanides totaux du jus désacidifié selon le procédé de l'invention ont été dosés selon la méthode Multi-laboratory validation of standard method for quantifying proanthocyanidins in cranberry powders, Wiley Interscience, 2010, et les résultats sont présentés dans le tableau suivant en équivalent proanthocyandines dimère A2.

| Échantillon | Proanthocyanides totaux (Eq. Dimer A2) mg/L |
|---|---|
| Jus de canneberge à désacidifier | 159.2 |
| Jus de canneberge désacidifié | 159.8 |

Le balayage des constituants (identifiés dans la table plus bas) a été effectué à l'aide d'un système de chromatographie liquide et de spectrométrie de masse à masse exacte (UPLC-QTOF) en mode négatif. Les échantillons ont été filtrés. Après traitement informatique, les masses exactes obtenues ont été regroupées par familles après identification à l'aide de banque de données. Le tableau suivant présente la somme des aires obtenues de l'ensemble des familles.

| Familles | Jus de canneberge non-traité | | Jus de canneberge traité | |
|---|---|---|---|---|
| | Aire | % (total) | Aire | % (total) |
| Proanthocyanidines | 2941 | 8.8 | 4164 | 9.5 |
| Acides phénoliques | 20315 | 61.0 | 26266 | 60.1 |
| Flavonoides | 10063 | 30.2 | 13277 | 30.4 |
| Total | 33319 | 100.0 | 43708 | 100.0 |

### Balayage des constituants

| Acides phénoliques | Proanthocyanidines | Flavonoides |
|---|---|---|
| Acide 2-hydroxybenzoique | (-)-Epicatechin | 3-Hydroxyphloretin 2'-O-glucoside |
| Acide 3-hydroxybenzoique | (-)-Epigallocatechin | Apigenine |
| 3-Méthyle cathecol | (+)-Catechin | Apigenine 6-C-glucoside |
| 4,6,3',4'-Tetraméthoxyaurone | (+)-Catechin 3-O-gallate | Apigenine 7-O-glucoronide |
| 4-Hydroxybenzaldéhyde | (+)-Catechin 3-O-glucose | Dihydromyricetin 3-O-rhamnoside |
| Acide 4-hydroxybenzoique | PAC A2 | Dihydroquercetin |
| 4-Hydroxycoumarin | PAC B2 | Fisatin |
| Acide 4-hydroxyphényle acétique | Trimers A | Isorhamnetin |
| 4-Vinylguaiacol | Trimers B | Isorhamnetin 3-O-glucoronide |
| 4-Vinylphénol | | Kaempferol-glucoside/galactoside |
| Acide 5,5'-déhydrodiférulique | | Kaempferol-glucoronide |
| Acide 5-O-galloylquinique | | Méthyl quercetin |
| Acide benzoique | | Myricetin |
| Acide cafféique | | Myricetin 3-O-arabinoside |
| Cafféoyl glucose | | Myricetin glucoside/ galactoside |
| Cathecol | | Phloretin |
| Acide chlorogénique | | Quercetin |
| Acide cinnamique | | Quercetin 3-O-arabinoside |
| Cinnamoyl glucose | | Quercetin 3-O-glucoronide |
| Coumarin | | Quercetin 3-O-xyloside |
| Coumaroyl glucoside | | Quercetin- glucoside/galactoside |
| Acide dihydro-p-coumarique | | Resveratrol |
| Acide ellagique | | Resveratrol-3-O-glucoside |
| Acide ellagique acétyl-arabinoside | | Rutin |
| Ferruloyl glucoside | | |
| Acide gallique | | |
| Acide gallique 3-O-gallate | | |
| Galloyl glucose | | |
| Acide hydroxycafféique | | |
| Acide m-coumarique | | |
| Acide o-coumarique | | |
| p-Anisaldéhyde | | |
| Acide p-coumarique | | |
| Acide p-coumarique éthyle ester | | |
| p-HPEA-EA | | |
| Acide protocatechuique | | |
| Ptérostilbène | | |
| Pyréthine II | | |
| Pyrogallol | | |
| Sinapaldéhyde | | |
| Acide sinapique | | |
| Acide syringique | | |
| Syringine | | |
| Acide vanilique | | |

Ce qui ressort clairement de la caractérisation est que contenu en composés d'intérêts comme les proanthocyanides, acides phénoliques, flavonoides et certains cations important du jus de canneberge désacidifié est substantiellement le même que jus de canneberge à désacidifier.

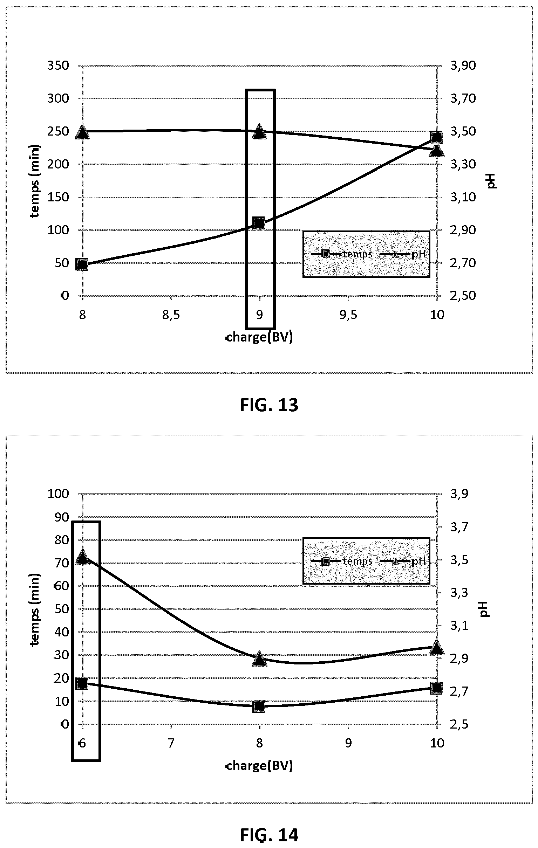

La figure 13 est un graphique représentant la variation de la durée de l'expérimentation, du pH à l'issue de l'expérimentation en fonction de la charge de jus de canneberge à désacidifier pour les 5 expérimentations réalisées avec la résine 1.

Au vu du graphique de la figure 13, on relève que la charge optimale de jus de canneberge à désacidifier lorsque la colonne est remplie de la résine 1 est 9 BV.

La figure 14 est un graphique représentant la variation de la durée de l'expérimentation, du pH à l'issue de l'expérimentation en fonction de la charge de jus de canneberge à désacidifier pour les 3 expérimentations réalisées avec la résine 2.

Au vu de ce graphique de la figure 14, on relève que la charge optimale de jus de canneberge à désacidifier lorsque la colonne est remplie de la résine 2 est 6 BV.

Pour la 3^{ième} expérimentation avec la résine 1, la saturation de la résine a été effectuée avec 2BV d'acide chlorhydrique (1 mol/L - 73g/L de résine) à un débit de 2 BV/heure selon un mode ascendant (« up flow »), suivie d'un rinçage lent avec 2 BV d'eau à un débit 2 BV/heure selon un mode ascendant (« up flow »).

Au cours de la saturation, les acides fixés par la résine sont libérés par le passage d'acide chlorhydrique.

Le tableau 20 ci-dessous détaille les concentrations en chlorures, acide malique et quinique dans l'effluent récupéré à la sortie de la résine 1, et ce en fonction du BV d'acide chlorhydrique, puis d'eau ayant circulé dans la résine 1 pour respectivement la saturer et la rincer. Le BV est exprimé de manière cumulée. Cela permet ainsi de suivre l'évolution de la concentration en chlorure, acides malique et quinique en fonction de l'avancée de la saturation de la résine 1, puis de son rinçage.

**Tableau 20 détaillant les concentrations en chlorures, acides malique et quinique au cours de la saturation en acide chlorhydrique, puis du rinçage de la résine 1**

| phase | BV | Concentration en chlorures (g/L) | Concentration en acide malique g/L | Concentration en acide quinique g/L |
|---|---|---|---|---|
| Acide chlorhydrique | 0,5 | 0,00 | 0,10 | 0,67 |
| | 1 | 0,00 | 0,10 | 0,63 |
| | 1,5 | 0,00 | 0,16 | 0,86 |
| | 2 | 0,00 | 8,56 | 2,95 |
| Eau | 2,5 | 0,00 | 15,48 | 2,41 |
| | 3 | 1,10 | 16,49 | 2,22 |
| | 3,5 | 0,50 | 9,83 | 1,47 |
| | 4 | 0,30 | 4,77 | 0,78 |

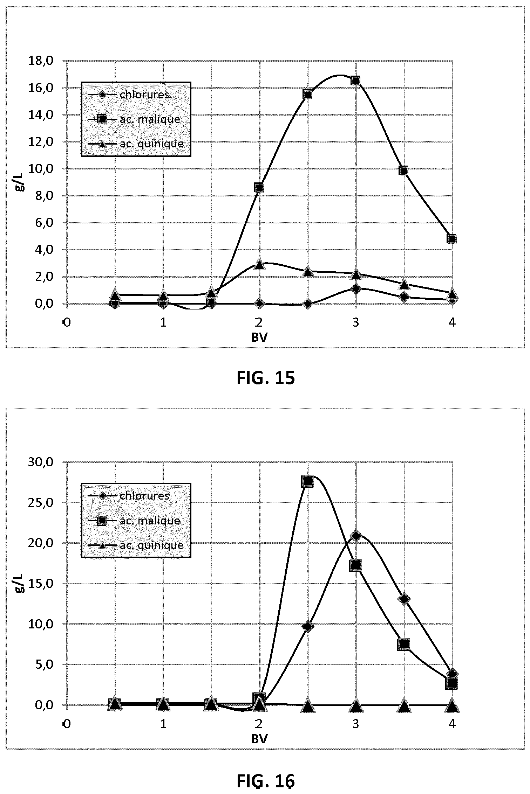

La figure 15 est un graphique de l'évolution de la concentration en chlorures, acide malique et acide quinique en fonction du BV cumulé d'acide chlorhydrique, puis d'eau.

Pour la 1^{ière} expérimentation avec la résine 2, la saturation de la résine a été effectuée avec 2BV d'acide chlorhydrique (lmol/L ; 73g/L de résine) à un débit de 2 BV/heure selon un mode ascendant (« up flow »), suivie d'un rinçage lent avec 2 BV d'eau à un débit 2 BV/heure selon un mode ascendant (« up flow »).

Le tableau 21 ci-dessous détaille les concentrations en chlorures, acide malique et quinique dans l'effluent récupéré à la sortie de la résine 2, et ce en fonction du BV d'acide chlorhydrique, puis d'eau ayant circulé dans la résine 2 pour respectivement la saturer et la rincer. Le BV est exprimé de manière cumulée.

**Tableau 21 détaillant les concentrations en chlorures, acides malique et quinique au cours de la saturation en acide chlorhydrique, puis du rinçage de la résine 2**

| phase | BV | Concentration en chlorures (g/L) | Concentration en acide malique g/L | Concentration en acide quinique g/L |
|---|---|---|---|---|
| Acide chlorhydrique | 0,5 | 0,00 | 0,17 | 0,27 |
| | 1 | 0,00 | 0,15 | 0,22 |
| | 1,5 | 0,00 | 0,14 | 0,20 |
| | 2 | 0,00 | 0,90 | 0,17 |
| Eau | 2,5 | 9,70 | 27,70 | 0,00 |
| | 3 | 20,90 | 17,30 | 0,00 |
| | 3,5 | 13,10 | 7,53 | 0,00 |
| | 4 | 3,80 | 2,73 | 0,00 |

La figure 16 est un graphique de l'évolution de la concentration en chlorures, acide malique et acide quinique en fonction du BV cumulé d'acide chlorhydrique, puis d'eau.

Au vu des tableaux 20 et 21 et des figures 15 et 16, on relève que :
- la résine 1 qui a une capacité d'échange plus importante que la résine 2 ne présente pratiquement pas d'excès de chlorures dans la fraction récupérée, alors qu'on mesure en moyenne 10 g/L de chlorures résiduel dans la fraction récupérée avec la résine 2. Environ 1/3 des chlorures n'est pas utilisé lors de la saturation avec la résine 2.

### D - EXEMPLE DE FORMULATIONS DE SIROP DE GRENADINE

Le tableau 22 détaille ci-dessous :
- la formulation d'un sirop de grenadine contenant du jus de canneberge désacidifié avec le procédé selon l'invention, c'est-à-dire une « formulation selon l'invention » ;
- la formulation d'un sirop de grenadine équivalente qui est classiquement mise en œuvre, c'est-à-dire une «formulation comparative ».

**Tableau 22 détaillant les formulations de sirop de grenadine selon l'invention et comparative**

| | Formulation comparative (kg) | Formulation invention (kg) |
|---|---|---|
| Sirop d'isoglucose à 65/70 °Bx | 3000 | 2700 |
| Jus de canneberge à 55 °Bx désacidifié selon l'invention | 0 | 300 |
| Arôme de grenadine | 20 | 20 |
| Eau | 850 | 905 |
| Acide citrique en solution | 55 | 0 |
| Colorant E122 | 0,55 | 0 |
| Arôme de vanille | 1 | 0 |
| Colorant E124 | 0,55 | 0 |
| Masse totale | 3927,1 | 3926 |

L'acide citrique était en solution dans de l'eau à une concentration de 150 g/L.

La formulation de sirop de grenadine selon l'invention présente les avantages par rapport à la formulation équivalente comparative d'être dépourvue de tout colorant, ainsi que d'acide citrique qui est un additif alimentaire utilisé comme correcteur d'acidité mais aussi connu pour être la cause de problèmes dentaires.

### E-EXEMPLE DE FORMULATIONS DE SIROP DE CANNEBERGE/FRAMBOISE

Le tableau 23 détaille ci-dessous :
- la formulation d'un sirop de canneberge et de framboise contenant du jus de canneberge désacidifié avec le procédé selon l'invention, c'est-à-dire une « formulation selon l'invention » ;
- la formulation d'un sirop de canneberge/framboise équivalente qui est classiquement mise en œuvre, c'est-à-dire une «formulation comparative ».

**Tableau 23 détaillant les formulations de sirop de canneberge/framboise selon l'invention et comparative**

| | Formulation comparative (kg) | Formulation invention (kg) |
|---|---|---|
| Jus de canneberge désacidifié selon l'invention | 0 | 513,3 |
| Jus de canneberge non désacidifié 65 °Bx | 14 | 14 |
| Sirop d'isoglucose 65 °Bx | 3000 | 2700 |
| Jus de raisin rouge 65 °Bx | 15 | 0 |
| Acide citrique en solution | 185 | 0 |
| Arôme de framboise et de canneberge | 8,6 | 8,6 |
| Colorant E124 | 0,3 | 0 |
| Jus de sureau 65 °Bx | 13 | 0 |
| Jus de framboise 65 °Bx | 15 | 15 |
| Masse totale | 3250,9 | 3250,9 |

La formulation du sirop de canneberge et de framboise selon l'invention, présente les avantages par rapport à la formulation équivalente comparative :
- d'être dépourvue de colorant E124 et d'acide citrique ;
- une partie du sucre du sirop d'isoglucose, ainsi que le sucre des sirops de sureau et de raisin ont été remplacés par le jus de canneberge désacidifié selon le procédé selon l'invention.

Il est à noter que la formulation comparative comprend du jus de canneberge non désacidifié.

Le fait d'avoir désacidifié le jus de canneberge avec le procédé selon l'invention permet de disposer d'une formulation de sirop de canneberge et de framboise qui comprend plus de jus de canneberge que la formulation équivalente comparative, et donc de mieux valoriser le jus de canneberge.

La formulation de sirop de canneberge et de framboise est perçue comme plus naturelle. Elle permet en outre de déclarer une teneur en jus de fruits rouges plus importante que la formulation comparative dont la teneur en fruits rouges consiste en la teneur en jus de canneberge non désacidifié additionnée à celle de l'arôme de framboise et de canneberge.

### F-FORMULATION DE SORBET DE CANNEBERGE

Le tableau 24 ci-dessous détaille une formulation de sorbet à base de canneberge désacidifié avec le procédé selon l'invention.

**Tableau 24 détaillant une formulation de sorbet à base de jus de canneberge désacidifié selon l'invention**

| | Quantité (g) |
|---|---|
| Jus de canneberge désacidifié 7,6 °Bx | 930 |
| Arômes naturels | 10 |
| Sirop de fructose 70 °Bx | 60 |
| Total | 1000 |

La faible acidité du jus de canneberge désacidifié avec le procédé selon l'invention permet de réaliser une formulation de sorbet contenant ce jus de canneberge en quantité importante (teneur massique 93% par rapport à la masse totale du sorbet). Les quantités de sirop de fructose et d'arômes naturels très faibles (respectivement des teneurs massiques 1% et 6% par rapport à la masse totale du sorbet), ainsi que l'absence de tout additif alimentaire et de colorant dans la formulation de sorbet témoignent d'un produit très naturel et "pur fruits ".

### G - FORMULATION DE SAUCE CULINAIRE DE TYPE BARBECUE

Dans cet exemple de produit salé, les notes fruitées de la canneberge sans ses inconvénients d'acidité et d'astringence sont associées à des notes traditionnelles de type barbecue, à savoir fumées, apportant un équilibre sucré/salé à la sauce.

Tous les ingrédients de la sauce détaillés dans le tableau 25 ci-dessous ont été mixés et thermisés à 85°C pendant 5 minutes afin de préserver la fraîcheur du produit qui a ensuite été pasteurisé et conditionné de manière aseptique.

**Tableau 25 détaillant une formulation de sauce culinaire de type barbecue à base de jus de canneberge désacidifié selon l'invention**

| | Quantité (g) |
|---|---|
| Jus de canneberge désacidifié 7,6 °Brix | 550 |
| Jus de canneberge désacidifié 55 °Brix | 255 |
| sel | 87 |
| Arômes naturels (oignon frit et épices) | 12 |
| Arômes fumés et viande (oignon frit et épices | 5 |
| Total | 1000 |

## Revendications

1. Jus de canneberge désacidifié **caractérisé en ce que** :
- le pH du jus désacidifié est compris entre 3,2 et 3.8,
- le ratio acide quinique /acide malique du jus désacidifié est d'environ 0.85 à environ 0.99 ;
- le jus désacidifié ne contient pas de sucres ajoutés, d'agent de masquage, tels une base ou un complexant, ou de tampon .

2. Jus de canneberge désacidifié selon la revendication 1, **caractérisé en ce que** le contenu en proanthocyanides totaux du jus de canneberge désacidifié ne varie pas à l'extérieur de la plage 95-105% du contenu en proanthocyanides totaux du jus de canneberge à désacidifier ayant conduit audit jus désacidifié.

3. Jus de canneberge désacidifié selon l'une quelconque des revendications 1 à 2, **caractérisé en ce que** le contenu en cations inorganique du jus de canneberge désacidifié ne varie pas à l'extérieur de la plage 95-105% du contenu en cations inorganique du jus de canneberge à désacidifier ayant conduit audit jus désacidifié.

4. Jus de canneberge désacidifié selon la revendication 1 **caractérisé en ce que** le contenu de deux, trois ou quatre des proanthocyanides, acides phénoliques, flavonoides et cations organiques dudit Jus de canneberge désacidifié n'est pas substantiellement inférieur au contenu du jus de canneberge à désacidifier.

5. Jus de canneberge désacidifié selon la revendication 3 ou 4, **caractérisé en ce que** les cations inorganique sont le potassium, le calcium et le sodium.

6. Procédé de désacidification d'un jus de canneberge à désacidifier, **caractérisé en ce que** :
- le jus de canneberge à désacidifier est élué sur une résine échangeuse d'anions faible pour conduire à un jus de canneberge désacidifié après l'élution;
- la résine échangeuse d'anions faibles est une résine de type acrylique ou de type styrénique ;
- le jus de canneberge à désacidifier est élué sur ladite résine à un taux (BV/h) tel que le jus de canneberge désacidifié après l'élution présente un pH : pKa₁ (acide malique) < pH < pKa (acide benzoique) ;
où « pKa₁ (acide malique) » représente le pKa de la 1^{ière} acidité de l'acide malique, et « pKa (acide benzoique) » représente le pKa de l'acide benzoique.

7. Procédé selon la revendication 6, **caractérisé en ce que** la résine échangeuse d'anions est de type acrylique.

8. Procédé selon la revendication 7, **caractérisé en ce que** la résine échangeuse d'anions de type acrylique possède une capacité entre 1.6-3.2 équivalent/L.

9. Procédé selon la revendication 7 ou 8, **caractérisé en ce que** la résine présente une vitesse d'échange initiale égale ou supérieure à +0,10 unité de pH/minute observée après 5 minutes de contact du jus à désacidifier avec la résine dans un ratio volumique de 5 :1 du jus à désacidifier pour la résine.

10. Procédé selon l'une quelconque des revendications 6 à 9, **caractérisé en ce qu'**il comprend en outre une étape de prétraitement consistant en une clarification du jus jusqu'à l'obtention d'une turbidité inférieure à 500 NTU.

11. Procédé selon l'une quelconque des revendications 6 à 10, caractérisé en ce le procédé de désacidification est réalisé dans une colonne contenant la résine échangeuse d'anions, et ladite élution consiste à faire circuler au moins une fois ledit jus de canneberge à désacidifier.

12. Procédé selon la revendication 11, **caractérisé en ce que** le taux d'élution du jus à désacidifier dans la colonne est égale ou supérieur à 10 BV/heure, comme par exemple dans la plage de 10 BV/heure à 250 BV/heure .

13. Procédé selon la revendication 11 ou 12, **caractérisé en ce que** le volume de jus de canneberge à désacidifier est d'environ 1 à 20 BV.

14. Composition alimentaire, **caractérisée en ce qu'**elle comprend du jus de canneberge selon l'une quelconque des revendications 1 à 5 ou désacidifié selon le procédé de l'une quelconque des revendications 6 à 13.

15. Composition alimentaire selon la revendication 14 pour son utilisation dans la prévention des infections urinaires.

## Patentansprüche

1. Entsäuerter Cranberrysaft, **dadurch gekennzeichnet, dass**:
- der pH-Wert des entsäuerten Saftes zwischen 3,2 und 3,8 beträgt,
- das Verhältnis Chinasäure / Apfelsäure des entsäuerten Saftes ungefähr 0,85 zu ungefähr 0,99 beträgt;
- der entsäuerte Saft keine Zuckerzusätze, kein Maskierungsmittel, wie eine Base oder einen Komplexbildner, oder keinen Puffer enthält.

2. Entsäuerter Cranberrysaft nach Anspruch 1, **dadurch gekennzeichnet, dass** der Gesamtgehalt an Proanthocyanidinen des entsäuerten Cranberrysaftes nicht außerhalb des Bereichs von 95-105 % des Gesamtgehalts an Proanthocyanidinen des zu entsäuernden Cranberrysaftes variiert, der zu dem entsäuerten Cranberrysaft geführt hat.

3. Entsäuerter Cranberrysaft nach einem der Ansprüche 1 bis 2, **dadurch gekennzeichnet, dass** der Gehalt an anorganischen Kationen des entsäuerten Cranberrysaftes nicht außerhalb des Bereichs von 95-105 % des Gehalts an anorganischen Kationen des zu entsäuernden Cranberrysaftes variiert, der zu dem entsäuerten Cranberrysaft geführt hat.

4. Entsäuerter Cranberrysaft nach Anspruch 1, **dadurch gekennzeichnet, dass** der Gehalt von zwei, drei oder vier der Proanthocyanidine, Phenolsäuren, Flavonoide und organischen Kationen des entsäuerten Cranberrysaftes nicht wesentlich geringer ist als der Gehalt des zu entsäuernden Cranberrysaftes.

5. Entsäuerter Cranberrysaft nach Anspruch 3 oder 4, **dadurch gekennzeichnet, dass** die anorganischen Kationen Kalium, Kalzium und Natrium sind.

6. Verfahren zur Entsäuerung eines zu entsäuernden Cranberrysaftes, **dadurch gekennzeichnet, dass**:
- der zu entsäuernde Cranberrysaft auf einem schwachen Anionenaustauscherharz eluiert wird, um zu einem entsäuerten Cranberrysaft nach der Elution zu führen;
- das schwache Anionenaustauscherharz ein Harz vom Typ Acrylharz oder vom Typ Styrolharz ist;
- der zu entsäuernde Cranberrysaft auf dem Harz mit einer Rate (BV/h) eluiert wird, so dass der entsäuerte Cranberrysaft nach der Elution folgenden pH-Wert aufweist: pKa₁ (Apfelsäure) < pH < pKa (Benzoesäure);
wobei "pKa₁ (Apfelsäure)" den pKa des 1. Säuregehalts der Apfelsäure darstellt und "pKa (Benzoesäure)" den pKa der Benzoesäure darstellt.

7. Verfahren nach Anspruch 6, **dadurch gekennzeichnet, dass** das Anionenaustauscherharz vom Typ Acrylharz ist.

8. Verfahren nach Anspruch 7, **dadurch gekennzeichnet, dass** das Anionenaustauscherharz vom Typ Acrylharz eine Volumenkapazität zwischen 1,6-3,2 Äquivalent/L besitzt.

9. Verfahren nach Anspruch 7 oder 8, **dadurch gekennzeichnet, dass** das Harz eine anfängliche Austauschgeschwindigkeit von gleich oder größer +0,10 pH-Einheit/Minute aufweist, beobachtet nach 5-minütigem Kontakt des zu entsäuernden Cranberrysaftes mit dem Harz in einem Volumenverhältnis von 5:1 des zu entsäuernden Cranberrysaftes zum Harz.

10. Verfahren nach einem der Ansprüche 6 bis 9, **dadurch gekennzeichnet, dass** es des Weiteren einen Vorbehandlungsschritt umfasst, der in einer Klärung des Saftes besteht, bis eine Trübung von weniger als 500 NTU erhalten wird.

11. Verfahren nach einem der Ansprüche 6 bis 10, **dadurch gekennzeichnet, dass** das Verfahren zur Entsäuerung in einer Kolonne ausgeführt wird, die das Anionenaustauscherharz enthält, und die Elution darin besteht, dass man den zu entsäuernden Cranberrysaft mindestens einmal zirkulieren lässt.

12. Verfahren nach Anspruch 11, **dadurch gekennzeichnet, dass** die Elutionsrate des zu entsäuernden Cranberrysaftes in der Kolonne gleich oder größer ist als 10 BV/Stunde, wie zum Beispiel in dem Bereich von 10 BV/Stunde bis 250 BV/Stunde.

13. Verfahren nach Anspruch 11 oder 12, **dadurch gekennzeichnet, dass** das Volumen des zu entsäuernden Cranberrysaftes ungefähr 1 bis 20 BV beträgt.

14. Lebensmittelzusammensetzung, **dadurch gekennzeichnet, dass** sie Cranberrysaft nach einem der Ansprüche 1 bis 5 oder entsäuert gemäß dem Verfahren nach einem der Ansprüche 6 bis 13 umfasst.

15. Lebensmittelzusammensetzung nach Anspruch 14 für ihre Verwendung in der Prävention von Harnwegsinfektionen.

## Claims

1. A deacidified cranberry juice **characterized in that**:
- the pH of the deacidified juice is comprised between 3.2 and 3.8;
- the quinic acid/malic acid ratio of the deacidified juice is about 0.85 to about 0.99;
- the deacidified juice does not contain added sugars, masking agents, such as basic agent or complexing agent, or a buffer.

2. The deacidified cranberry juice according to claim 1, **characterized in that** the total proanthocyanidins content of the deacidified cranberry juice does not vary outside the range 95-105% of the total proanthocyanidins content of the cranberry juice to be deacidified leading to said deacidified juice.

3. The deacidified cranberry juice according to any one of claims 1 to 2, **characterized in that** the inorganic cation content of the deacidified cranberry juice does not vary outside the range 95-105% of the inorganic cation content of the cranberry juice to be deacidified leading to said deacidified juice.

4. The deacidified cranberry juice according to claim 1, **characterized in that** the content of two, three or four of the proanthocyanidins, phenolic acids, flavonoids and organic cations of said deacidified cranberry juice is not substantially less than the content of the cranberry juice to be deacidified.

5. The deacidified cranberry juice according to claim 3 or 4, **characterized in that** the inorganic cations are the potassium, the calcium and the sodium.

6. A method for deacidifying a cranberry juice to be deacidified, **characterized in that**:
- the cranberry juice to be deacidified is eluted on a weak anion exchange resin to lead to a deacidified cranberry juice after elution;
- the weak anion exchange resin is a resin of the acrylic type or styrenic type;
- the cranberry juice to be deacidified is eluted on said resin at a rate (BV/h) such as the deacidified cranberry juice after elution has a pH: pKa₁ (malic acid) < pH < pKa (benzoic acid);
where « pKa₁ (malic acid) » represents the pKa of the 1^{st} acidity of the malic acid, and « pKa (benzoic acid) » represents the pKa of the benzoic acid.

7. The method according to claim 6, **characterized in that** the anion exchange resin is of the acrylic type.

8. The method according to claim 7, **characterized in that** the anion exchange resin of the acrylic type has a capacity between 1.6 - 3.2 equivalent/L.

9. The method according to claim 7 or 8, **characterized in that** the resin has an initial exchange rate equal to or greater than +0.10 pH unit/minute observed after 5 minutes of the juice to be deacidified has been in contact with the resin in a volume ratio of 5:1 of the juice to be deacidified for the resin.

10. The method according to any one of claims 6 to 9, **characterized in that** it further comprises a pre-treatment step consisting of a clarification of the juice until a turbidity less than 500 NTU is obtained.

11. The method according to any one of claims 6 to 10, **characterized in that** the deacidification method is carried out in a column containing the anion exchange resin, and said elution consists of circulating at least once said cranberry juice to be deacidified.

12. The method according to claim 11, **characterized in that** the elution rate of the juice to be deacidified in the column is equal to or greater than 10 BV/hour, such as in the range of 10 BV/hour to 250 BV/hour.

13. The method according to claim 11 or 12, **characterized in that** the volume of cranberry juice to be deacidified is about 1 to 20 BV.

14. A food composition **characterized in that** it comprises cranberry juice according to any one of claims 1 to 5 or deacidified juice according to the method of any one of claims 6 to 13.

15. The food composition according to claim 14 for its use in the prevention of urinary tract infections.
